# EUROPÄISCHE PATENTSCHRIFT

(11) **EP 0 891 330 B1**
(45) Veröffentlichungstag und Bekanntmachung des Hinweises auf die Patenterteilung: **08.03.2006**
(21) Anmeldenummer: 97915409.3
(22) Anmeldetag: 21.03.1997
(51) Int. Cl.: C07D 209/54, A01N 43/00, C07D 491/10, C07D 207/38, C07D 307/94, C07D 333/50, C07D 309/32, C07D 405/04

(54) **SUBSTITUIERTE PHENYLKETOENOLE ALS SCHÄDLINGSBEKÄMPFUNGSMITTEL UND HERBIZIDE**
SUBSTITUTED PHENYL KETO ENOLS AS PESTICIDES AND HERBICIDES
PHENYLCETO-ENOLS SUBSTITUES UTILISES COMME PESTICIDES ET HERBICIDES

(30) Priorität: 02.04.1996 DE 19613171; 29.11.1996 DE 19649665
(43) Veröffentlichungstag der Anmeldung: 20.01.1999
(60) Teilanmeldung: 05026674.1
(73) Patentinhaber: Bayer CropScience AG, 40789 Monheim (DE)
(72) Erfinder: LIEB, Volker, D-51375 Leverkusen (DE); HAGEMANN, Hermann, D-51375 Leverkusen (DE); WIDDIG, Arno, D-51519 Odenthal (DE); RUTHER, Michael, D-40789 Monheim (DE); FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); GRAFF, Alan, D-51061 Köln (DE); SCHNEIDER, Udo, D-51373 Leverkusen (DE)
(86) Internationale Anmeldenummer: PCT/EP1997/001426
(87) Internationale Veröffentlichungsnummer: WO 1997/036868

(56) Entgegenhaltungen:
- EP-A- 0 456 063
- EP-A- 0 521 334
- EP-A- 0 613 885
- WO-A-95/26954
- DE-A- 4 216 814
- DE-A- 4 326 909
- DE-A- 4 337 853
- DE-A- 4 410 420
- DE-A- 4 413 669
- DE-A- 4 425 617
- DE-A- 19 602 524
- DE-A- 19 603 332

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In FP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, DE 44 40 594, WO 94/01 997, WO 95/01 358, WO 95/20 572, EP-A-668 267 und WO 95/26 954.

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-△³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschatten aus EP-A-528 156 und EP-A-0 647 637 bekannt. Auch 3-Aryl-Δ³-dihydrothiophen-on-Derivate sind bekannt (WO 95/26 345).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die akarizide und insektizide Wirksamkeit und/oder Wirkungsbreite, und/oder die Pflanzenverträglichkeit dieser Verbindungen, insbesondere gegenüber Kulturpflanzen, ist jedoch nicht immer ausreichend.

Es wurden nun neue Verbindungen der Formel (I) 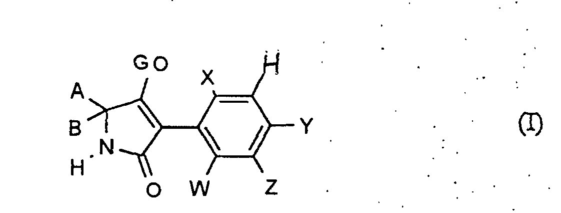 in welcher
- W: für Halogen oder C₁-C₆-Alkyl, steht,
- X: für Halogen, C₁-C₆ Alkyl oder C₁-C₆-Alkoxy, steht,
- Y: für Wasserstoff, Halogen oder C₁-C₆-Alkyl, steht,
- Z: für Halogen oder C₁-C₆-Alkyl, steht,
- A: für C₁-C₁₂-Alkyl, steht,
- B: für C₁-C₁₂-Alkyl steht oder
- A, B: und das Kohlenstoffatom an das sie gebunden sind, für C₃-C₁₀₋Cycloalkyl stehen, welche gegebenenfalls durch C₁-C₈-Alkyl, oder C₁-C₈-Alkoxy, substituiert sind,
- G: für Wasserstoff (a) oder für eine der Gruppen 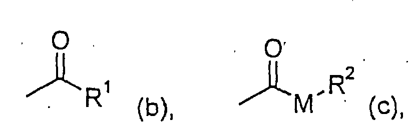
steht,
in welchen
M für Sauerstoff oder Schwefel steht,
- R¹: für jeweils gegebenenfalls durch Halogen substituiertes C₁-C₂₀₋Alkyl, C₂-C₂₀-Alkenyl, C₁-C₈-Alkoxy-C₁-C₈-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆₋Alkylthio oder C₁-C₆-Alkylsulfonyl substituiertes Phenyl,
für gegebenenfalls durch Halogen oder C₁-C₆-Alkyl substituiertes 5- oder 6-gliedriges Hetaryl mit ein oder zwei Heteroatomen aus der Reihe Sauerstoff, .Schwefel und Stickstoff,
- R²: für jeweils gegebenenfalls durch Halogen substituiertes C₁-C₂₀-Alkyl, C₂-C₂₀-Alkenyl, C₁-C₈-Alkoxy-C₂-C₈-alkyl,
für jeweils gegebenenfalls durch Halogen, Cyano, Nitro, C₁-C₆₋Alkyl, C₁-C₆-Alkoxy, C₁-C₆-Halogenalkyl oder C₁-C₆-Halogenalkoxy substituiertes Phenyl oder Benzyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) der Gruppe Het ergeben sich folgende hauptsächliche Strukturen (I-1): 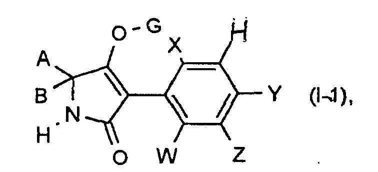
worin
A, B, G, W, X, Y und Z die oben angegebene Bedeutung haben.

Unter Einbeziehung der verschiedenen Bedeutungen (a), (b), (c), der Gruppe G ergeben sich folgende hauptsächliche Strukturen (I-1-a) bis (1-1-c), wenn Het für die Gruppe (1) steht, 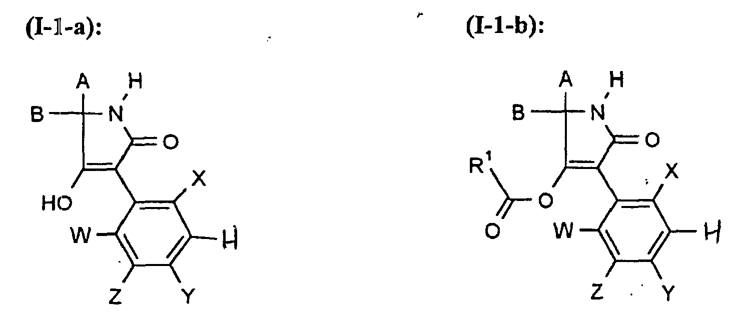 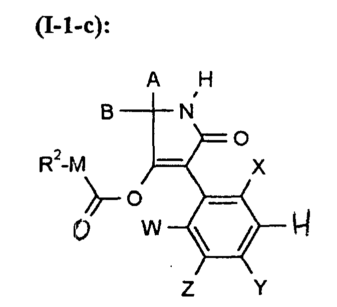
worin

- A, B, M, W, X, Y, Z, R¹, R²: die oben angegebenen Bedeutungen besitzen.

Weiterhin wurde gefunden, daß man die neuen Verbindungen der Formel (I) nach einem der im folgenden beschriebenen Verfahren erhält:
(A) Man erhält Verbindungen der Formel (I-1-a) 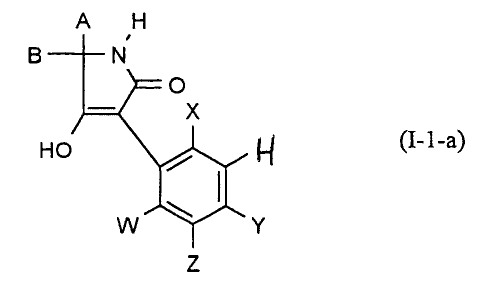
   in welcher
   - A, B, W, X, Y und Z: die oben angegebenen Bedeutungen haben,

   wenn man
   Verbindungen der Formel (II) 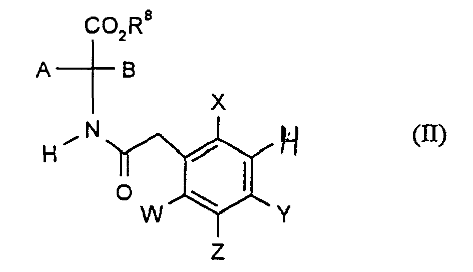
   in welcher
   - A, B, W, X, Y und Z: die oben angegebenen Bedeutungen haben,

   und
   - R⁸: für Alkyl (bevorzugt C₁-C₆-Alkyl) steht,

   in Gegenwart eines Verdünnungsmittels und in Gegenwart einer Base intramolekular kondensiert. Außerdem wurde gefunden,
(F) daß man die Verbindungen der oben gezeigten Formeln (I-1-b) in welchen A, B, R¹, W, X, Y und Z die oben angebenen Bedeutungen haben, erhält, wenn man Verbindungen der oben gezeigten Formeln (I-1-a), in welchen A, B, W, X, Y und Z die oben angegebenen Bedeutungen haben,
α) mit Säurehalogeniden der Formel (VIII) 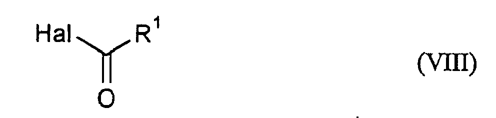
   in welcher
   - R¹: die oben angegebene Bedeutung hat und
   - Hal: für Halogen (insbesondere Chlor oder Brom) steht

   oder
β) mit Carbonsäureanhydriden der Formel (IX)

   R¹-CO-O-CO-R¹ (IX)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt
(G) daß man die Verbindungen der oben gezeigten Formeln (I-1-c) in welchen A, B, R², W, M, X, Y und Z die oben angegebenen Bedeutungen haben und L für Sauerstoff steht, erhält, wenn man Verbindungen der oben gezeigten Formeln (I-1-a) in welchen A, B, W, X, Y und Z die oben angegebenen Bedeutungen haben, jeweils
   mit Chlorameisensäureestern oder Chlorameisensäurethioestern der Formel (X)

   R²-M-CO-Cl (X)

   in welcher
   - R² und M: die oben angegebenen Bedeutungen haben,

   gegebenenfalls in Gegenwart eines Verdünnungsmittels und gegebenenfalls in Gegenwart eines Säurebindemittels umsetzt;

Weiterhin wurde überraschend gefunden, daß die neuen Verbindungen der Formel (I) eine sehr gute Wirksamkeit als Schädlingsbekämpfungsmittel, vorzugsweise als Insektizide, Akarizide und als Herbizide aufweisen und darüber hinaus sehr gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert: bevorzugt steht
- W: für Fluor, Chlor, Brom oder C₁-C₄-Alkyl steht,
- X: für Fluor, Chlor, Brom, C₁-C₄-Alkyl, oder C₁-C₄-Alkoxy, steht,
- Y: für Wasserstoff, Brom, Chlor, C₁-C₄-Alkyl, steht,
- Z: für Fluor, Chlor, Brom oder C₁-C4-Alkyl, steht,
- A: für C₁-C₁₀-Alkyl, steht,
- B: für C₁-C₁₀-Alkyl steht,
- A, B: und das Kohlenstoffatom an das sie gebunden sind für C₃-C₈-Cycloalkyl stehen, welche gegebenenfalls durch C₁-C₆-Alkyl; oder C₁-C₆-Alkoxy, substituiert sind
- D: für Wasserstoff steht
- G: für Wasserstoff (a) oder für eine der Gruppen 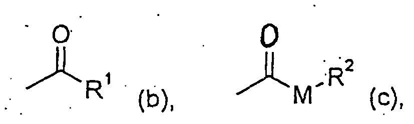
in welchen
M für Sauerstoff oder Schwefel steht.
- R¹: für jeweils gegebenenfalls durch Fluor oder Chlor substituiertes C₁₋C₁₆-Alkyl, C₂-C₁₆-Alkenyl, C₁-C₆-Alkoxy-C₁-C₆-alkyl, oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄₋Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für jeweils gegebenenfalls durch Fluor, Chlor, Brom oder C₁-C₄₋Alkyl substituiertes Pyrazolyl, Thiazolyl, Pyridyl, Pyrimidyl, Furanyl oder Thienyl,
- R₂: für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl,
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃₋Halogenalkoxy substituiertes Phenyl oder Benzyl steht.

Besonders bevorzugt steht worin
- V: für Wasserstoff steht,
- W: für Brom, Chlor oder Methyl, steht,
- X: für Brom, Chlor, Methyl oder Methoxy, steht,
- Y: für Wasserstoff, Brom, Chlor oder Methyl steht,
- Z: für Chlor, Brom, oder Methyl, steht,
- A: für C₁-C₈-Alkyl, steht,
- B: für C₁-C₈-Alkyl steht oder
- A, B: und das Kohlenstoffatom an das sie gebunden sind, für C₃-C₈₋Cycloalkyl stehen, welche gegebenenfalls durch Methyl, oder Trifluormethyl, Methoxy, Ethoxy, n-Propoxy, substituiert sind
- D: für Wasserstoff steht,
- G: für Wasserstoff (a) oder für eine der Gruppen 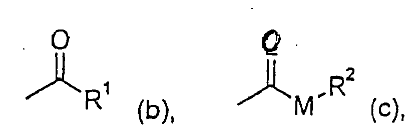
in welchen
M für Sauerstoff oder Schwefel steht.
- R¹: für jeweils gegebenenfalls durch Fluor oder Chlor substituiertes C₁-C₁₄-Alkyl; (C₂-C₁₄-Alkenyl, C₁-C₄-Alkoxy-C₁-C₆-alkyl, oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, , i-Propyl, Methoxy, Ethoxy, Trifluormethyl, Trifluormethoxy, Methylthio, Ethylthio, Methylsulfonyl oder Ethylsulfonyl substituiertes Phenyl,
für jeweils gegebenenfalls durch Fluor, Chlor, Brom, Methyl oder Ethyl substituiertes Furanyl, Thienyl oder Pyridyl,
Phenoxy-C₁-C₄-alkyl oder
- R²: für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Insbesondere bevorzugt sind Verbindungen der Formel I-1 in welchen
b) A, B und das Kohlenstoffatom, an das sie gebunden sind, die als ganz besonders bevorzugt genannten Bedeutungen haben und Y für Wasserstoff stehen.

Im einzelnen seien außer den bei den Herstellungsbeispielen genannten Verbindungen die folgenden Verbindungen der Formel (I-1-a) genannt:

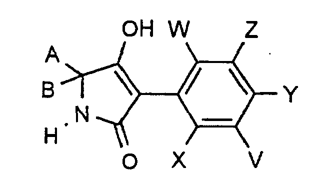

**Tabelle 1 V = H; W = CH₃; X = CH₃ ; Y = CH₃ ; Z = CH₃**

| A | B |
|---|---|
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| -(CH₂)₂- | |
| -(CH₂)₄- | |
| -(CH₂)₅- | |
| -(CH₂)₆- | |
| -(CH₂)₇- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |

- **Tabelle 2:**: A und B haben die gleiche Bedeutung wie in Tabelle 1 mit V = H; W = CH₃; X = CH₃; Y = H; Z = Cl
- **Tabelle 3:**: A und B haben die gleiche Bedeutung wie in Tabelle 1 mit V=H; W=CH₃; X=CH₃; Y=CH₃; Z=F
- **Tabelle 4:**: A und B haben die gleiche Bedeutung wie in Tabelle 1 mit V = H; W = CH₃; X = CH₃; Y = CH₃; Z = Cl
- **Tabelle 5:**: A und B haben die gleiche Bedeutung wie in Tabelle 1 mit V = H; W = CH₃; X = CH₃; Y = CH₃; Z = Br
- **Tabelle 8:**: A und B haben die gleiche Bedeutung wie in Tabelle 1 mit V = H; W = CH₃; X = CH₃; Y = H; Z = Br
- **Tabelle 9:**: A und B haben die gleiche Bedeutung wie in Tabelle 1 mit V=H;W=Cl;X=Cl;Y=H;Z=Br
- **Tabelle 10:**: A und B haben die gleiche Bedeutung wie in Tabelle 1 mit V = H; W = Br; X = Br; Y, Z = -(CH₂)₃-
- **Tabelle 11:**: A und B haben die gleiche Bedeutung wie in Tabelle 1 mit V=H;W=CH₃;X=OCH₃;Y=H;Z=Br
- **Tabelle 13:**: A und B haben die gleiche Bedeutung wie in Tabelle 1 mit V=H; W=Cl; X=Cl; Y=Cl; Z=CH₃
- **Tabelle 14:**: A und B haben die gleiche Bedeutung wie in Tabelle 1 mit V = H; W = Br; X = Br; Y = Br; Z = CH₃

Verwendet man gemäß Verfahren (A). N-[(3,4-Dichlor-2,6-dimethyl)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

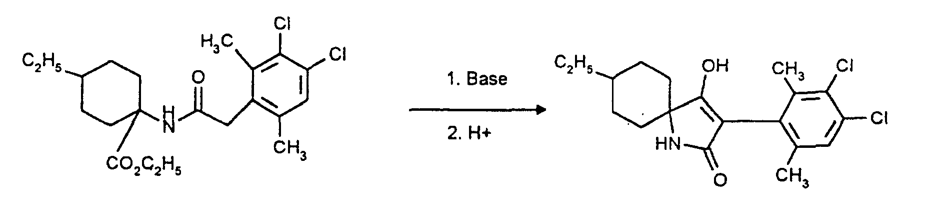

Verwendet man gemäß Verfahren (Fα) 3-[(2,5-Dichlor-4,6-dimethyl)-phenyl]-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

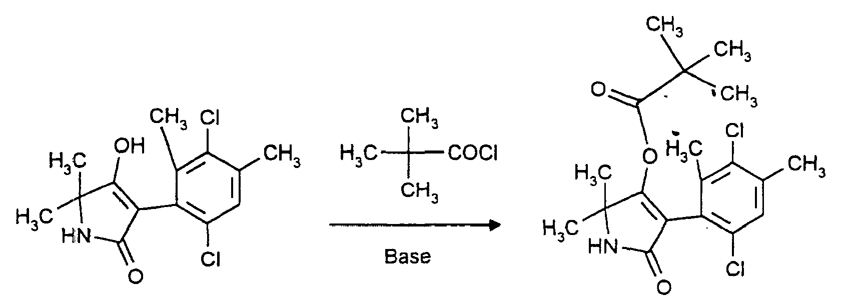

Verwendet man beispielsweise gemäß Verfahren (F) (Variante β) 3-[(2,3-Dichlor)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

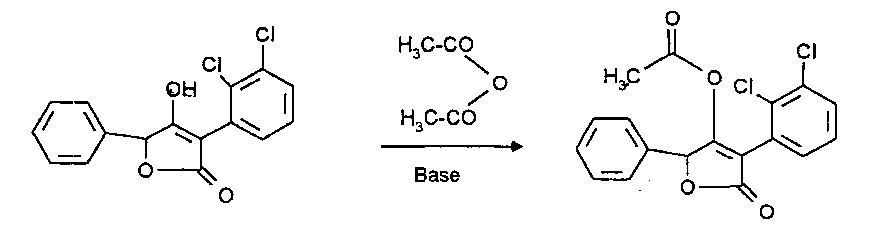

Verwendet man gemäß Verfahren (G) 8-[(2,4-Dichlor-3-methyl)-phenyl]-5,5-pentamethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

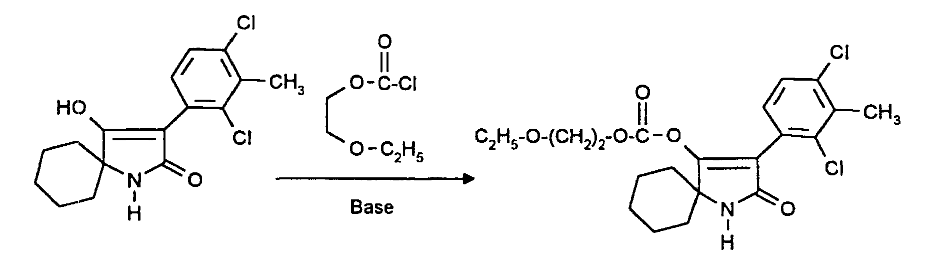

Die beim erfindungsgemäßen Verfahren (A) als Ausgangsstoffe benötigten Verbindungen der Formel (II) 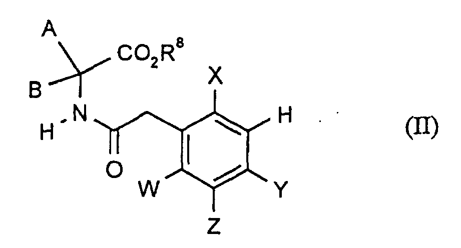
in welcher
- A, B, W, X, Y, Z und R⁸: die oben angegebenen Bedeutungen haben,

sind neu.

Man erhält die Acylaminosäureester der Formel (II) beispielsweise, wenn man Aminosäurederivate der Formel (XIX) 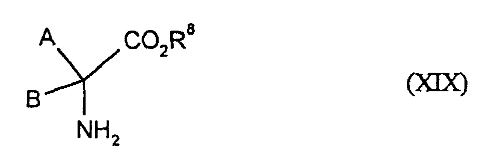
in welcher
- A, B und R⁸: die oben angegebenen Bedeutungen haben,

mit substituierten Phenylessigsäurehalogeniden der Formel (XX) 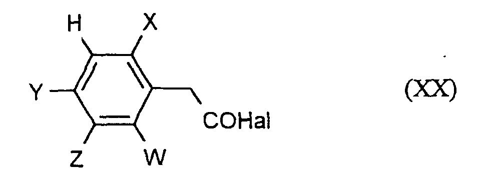
in welcher
- W, X, Y und Z: die oben angegebenen Bedeutungen haben und
- Hal: für Chlor oder Brom steht,

acyliert (Chem. Reviews 52, 237-416 (1953); Bhattacharya, Indian J. Chem. 6, 341-5, 1968)
oder wenn man Acylaminosäuren der Formel (XXI) 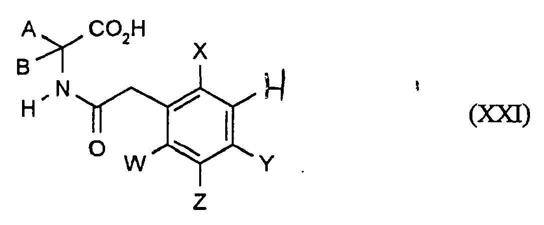
in welcher
- A, B, W, X, Y und Z: die oben angegebenen Bedeutungen haben,

verestert (Chem. Ind. (London) 1568 (1968)).

Die Verbindungen der Formel (XXI) 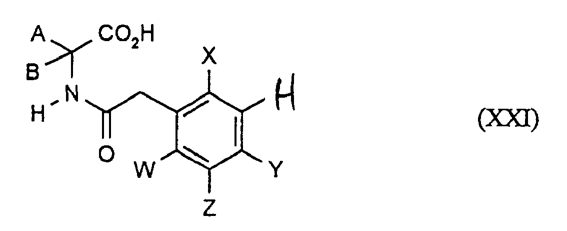
in welcher
- A, B, W, X, Y und Z: die oben angegebenen Bedeutungen haben,

sind neu.

Man erhält die Verbindungen der Formel (XXI), wenn man Aminosäuren der Formel (XXII) 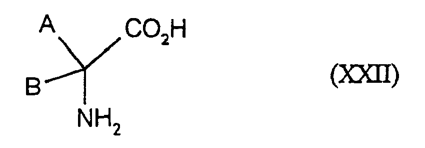
in welcher
- A und B: die oben angegebenen Bedeutungen haben,

mit substituierten Phenylessigsäurehalogeniden der Formel (XX) 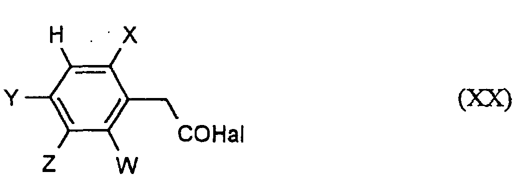
in welcher
- W, X, Y und Z: die oben angegebenen Bedeutungen haben und
- Hal: für Chlor oder Brom steht,

nach Schotten-Baumann acyliert (Organikum, VEB Deutscher Verlag der Wissenschaften, Berlin 1977, S. 505).

Die Verbindungen der Formel (XX) sind teilweise neu und lassen sich nach bekannten Verfahren herstellen.

Man erhält die Verbindungen der Formel (XX) beispielsweise, indem man substituierte Phenylessigsäuren der Formel (XXIII) 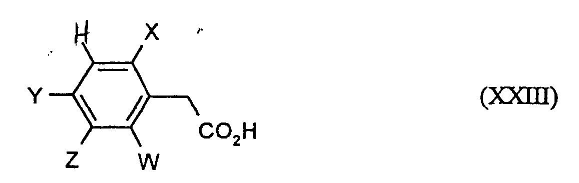
in welcher
- W, X, Y und Z: die oben angegebene Bedeutung haben,

mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) sind teilweise neu, sie lassen sich nach literaturbekannten Verfahren herstellen (Organikum 15. Auflage, S. 533, VEB Deutscher Verlag der Wissenschaften, Berlin 1977). Man erhält die Verbindungen der Formel (XXIII) beispielsweise, indem man substituierte Phenylessigsäureester der Formel (XXIV) 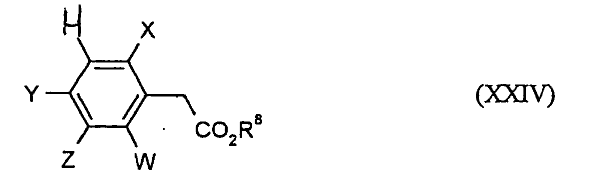
in welcher
- W, X, Y, Z und R⁸: die oben angegebene Bedeutung haben,

in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXIV) sind teilweise neu, sie lassen sich nach im Prinzip bekannten Verfahren herstellen.

Man erhält die Verbindungen der Formel (XXIV) beispielsweise, indem man substituierte 1,1,1-Trichlor-2-phenylethane der Formel (XXV) 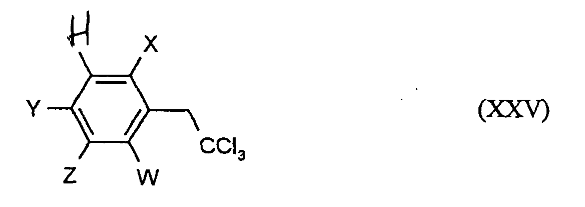
in welcher
- W, X, Y und Z: die oben angegebene Bedeutung haben,

zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE 3 314 249).

Die Verbindungen der Formel (XXV) sind teilweise neu, sie lassen sich nach im Prinzip bekannten Verfahren herstellen.

Man erhält die Verbindungen der Formel (XXV) beispielsweise, wenn man Aniline der Formel (XXVI) 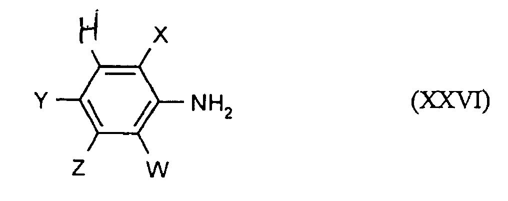
in welcher
- W, X, Y und Z: die oben angegebene Bedeutung haben,

in Gegenwart eines Alkylnitrits der Formel (XXVII)

R²¹-ONO (XXVII)

in welcher
- R²¹: für Alkyl, bevorzugt C₁-C₆-Alkyl steht,

in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XXVI) sind teilweise bekannte Verbindungen oder lassen sich nach im Prinzip bekannten Verfahren herstellen. Die Verbindungen der Formel (XXVII) sind bekannte Verbindungen der Organischen Chemie. Kupfer(II)-chlorid und Vinylidenchlorid sind lange bekannt und käuflich.

Die Verbindungen der Formel (XIX) und (XXII) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 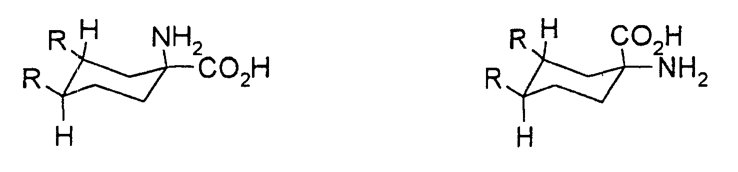 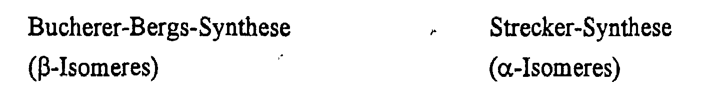
(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 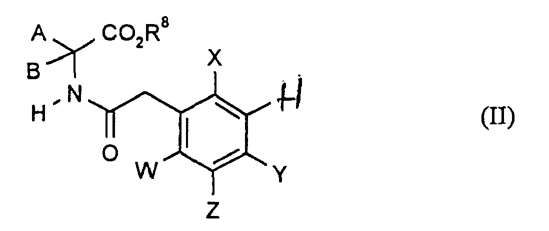
in welcher
- A, B, W, X, Y, Z und R⁸: die oben angegebenen Bedeutungen haben,

herstellen, wenn man Aminonitrile der Formel (XXVIII) 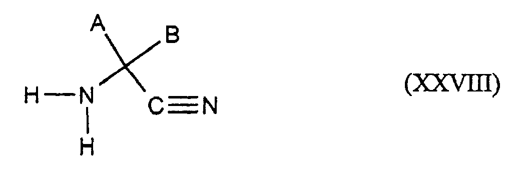
in welcher
- A und B: die oben angegebenen Bedeutungen haben,

mit substituierten Phenylessigsäurehalogeniden der Formel (XX) 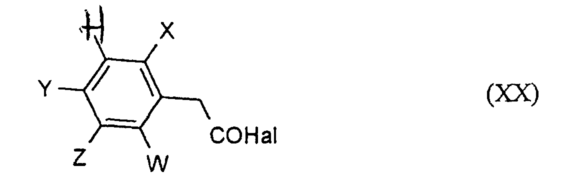
in welcher
- W, X, Y, Z und Hal: die oben angegebenen Bedeutungen haben,

zu Verbindungen der Formel (XXIX) 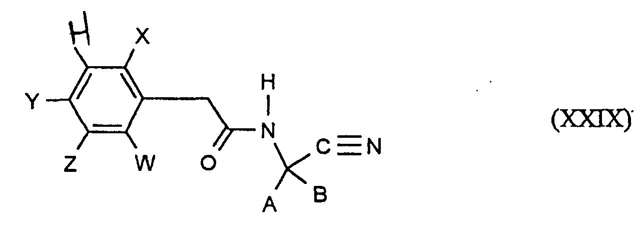
in welcher
- A, B, W, X, Y und Z: die oben angegebenen Bedeutungen haben,

umsetzt,
und diese anschließend einer sauren Alkoholyse unterwirft.

Die Verbindungen der Formel (XXIX) sind ebenfalls neu.

Die zur Durchführung der erfindungsgemäßen Verfahren (F), (G), und außerdem als Ausgangsstoffe benötigten Säurehalogenide der Formel (VIII), Carbonsäureanhydride der Formel (IX), Chlorameisensäureester oder Chlorameisensäurethioester der Formel (X), sind allgemein bekannte Verbindungen der organischen bzw. anorganischen Chemie.

Die Verbindungen der Formeln (VIII), (IX), (X), (XIX), (XXII), (XXVIII), sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -50°C und 250°C, vorzugsweise zwischen -30°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (Fα) ist dadurch gekennzeichnet, daß man Verbindungen der Formeln (I-1-a) jeweils mit Carbonsäurehalogeniden der Formel (VIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Fα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Fα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fα) werden die Ausgangsstoffe der Formeln (I-1-a) und das Carbonsäurehalogenid der Formel (VIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Fβ) ist dadurch gekennzeichnet, daß man Verbindungen der Formeln (I-1-a) jeweils mit Carbonsäureanhydriden der Formel (IX) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Fβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Fβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fβ) werden die Ausgangsstoffe der Formeln (I-1-a) und das Carbonsäureanhydrid der Formel (IX) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (G) ist dadurch gekennzeichnet, daß man Verbindungen der Formeln (I-1-a) jeweils mit Chlorameisensäureestern oder Chlorameisensäurethiolestern der Formel (X) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (G) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (G) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (G) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (G) wird im allgemeinen unter Normaldruck durchgeführt.

Bei der Durchführung des erfindungsgemäßen Verfahrens (G) werden die Ausgangsstoffe der Formeln (I-1-a) und der entsprechende Chlorameisensäureester bzw. Chlorameisensäurethiolester der Formel (X) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
   Aus der Ordnung der Mallophaga z.B. Trichodectes spp., Damalinea spp.
   Aus der Ordnung der Thysanoptera z.B. Hercinothrips femoralis, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und
synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyano-phenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Herstellungsbeispiele

### Beispiel (I-1-a-1)

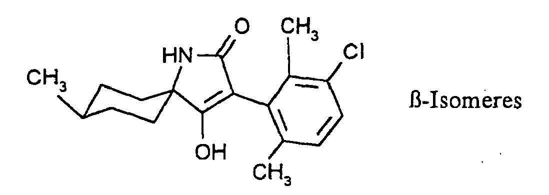

Zu 22,7 g (0,2 Mol) Kalium-tert.-butylat in 60 ml wasserfreiem Tetrahydrofuran (THF) tropft man bei Rückflußtemperatur 29,6 g (0,0764 Mol) der Verbindung gemäß Beispiel (II-1) in 160 ml wasserfreiem Toluol und rührt 1,5 Stunden unter Rückfluß. Zur Aufarbeitung gibt man 230 ml Wasser zu, trennt die wäßrige Phase ab, extrahiert die Toluolphase mit 110 ml Wasser, vereinigt die wäßrigen Phasen, wäscht sie mit Toluol und säuert bei 10 bis 20°C mit konz. HCl auf pH 1 an. Das Produkt wird abgesaugt, gewaschen, getrocknet und durch Verrühren in Methyl-tert.-butyl(MTB)-ether/n-Hexan gewaschen.
Ausbeute: 13,7 g (56 % der Theorie), Fp.: >220°C.
Analog bzw. gemäß den allgemeinen Angaben zur Herstellung erhält man die folgenden Verbindungen der Formel (I-1-a):

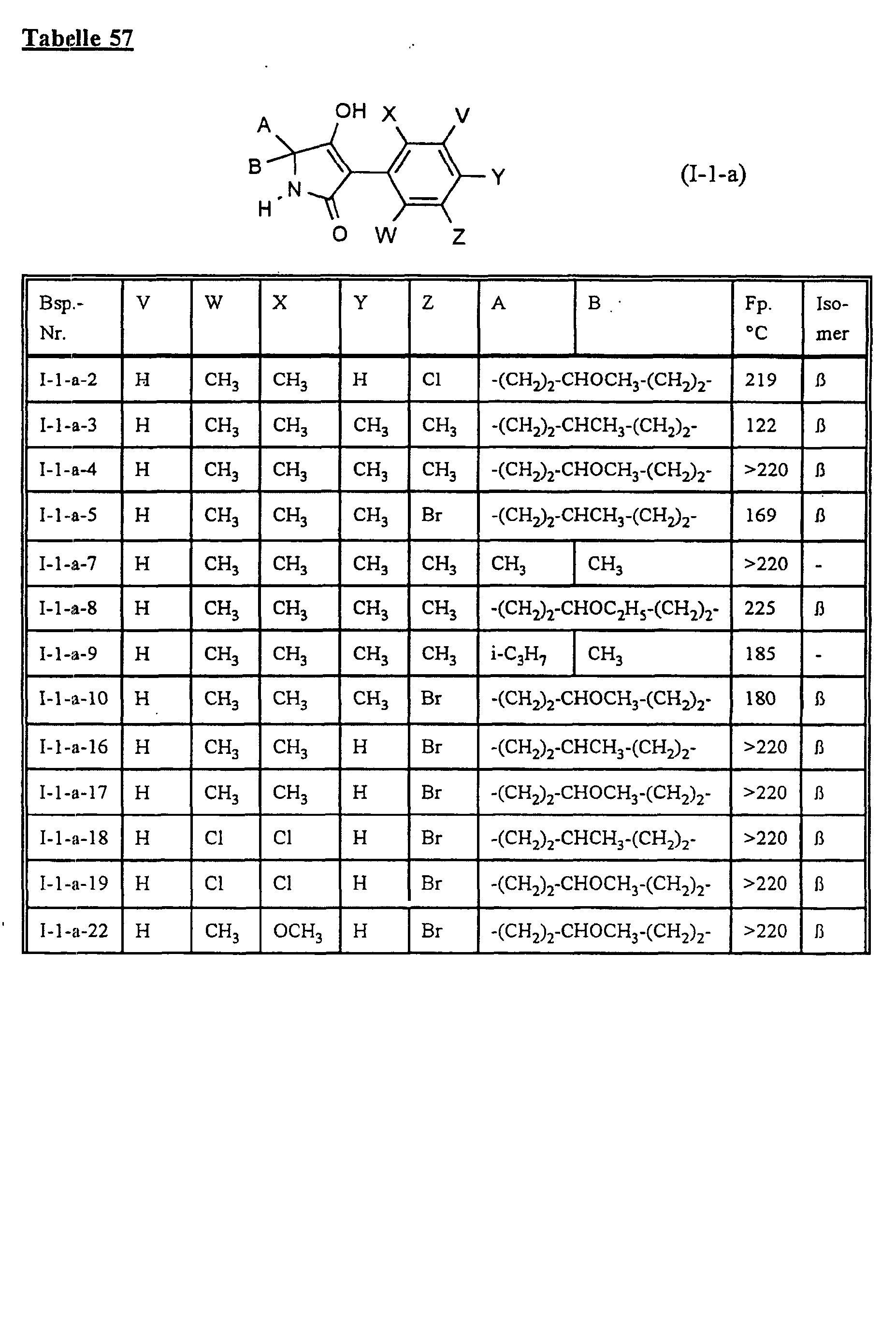

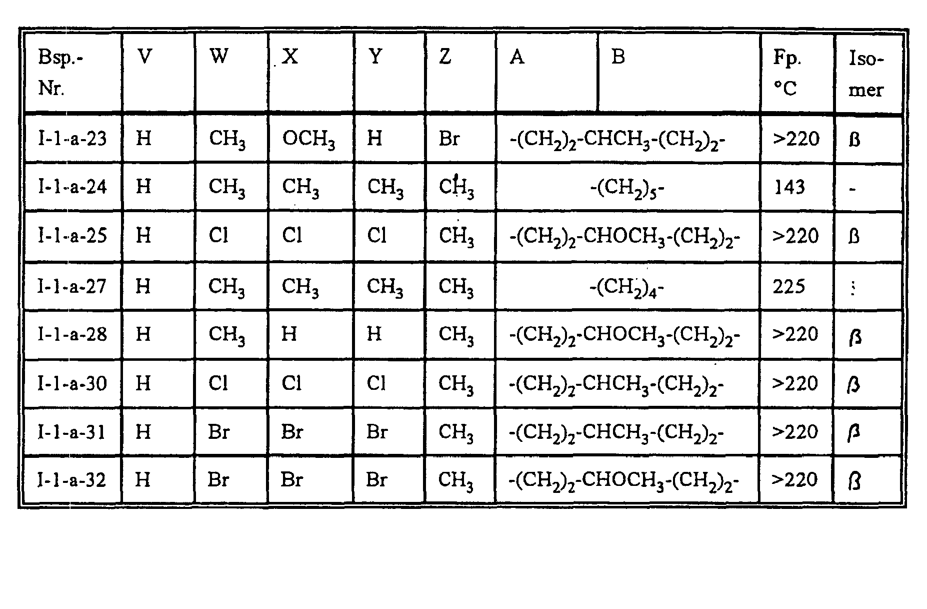

### Beispiel (I-1-b-1)

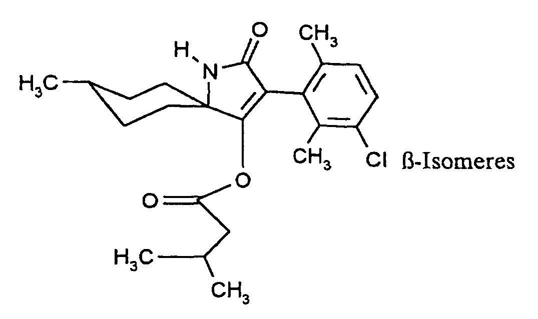

3,84 g (0,012 Mol) der Verbindung gemäß Beispiel (I-1-a-1) und 2,5 ml (18 mMol) Triethylamin in 70 ml wasserfreiem Methylenchlorid werden bei 0 bis 10°C mit 2,2 ml (0,18 Mol) Isovaleriansäurechlorid in 5 ml wasserfreiem Methylenchlorid versetzt. Bei Raumtemperatur wird gerührt, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft. Das Rohprodukt wird aus MTB-Ether/n-Hexan umkristallisiert.
Ausbeute: 1,6 g (33 % der Theorie), Fp.: 218°C.
Analog bzw. gemäß den allgemeinen Angaben zur Herstellung erhält man die folgenden Verbindungen der Formel (I-b-1):

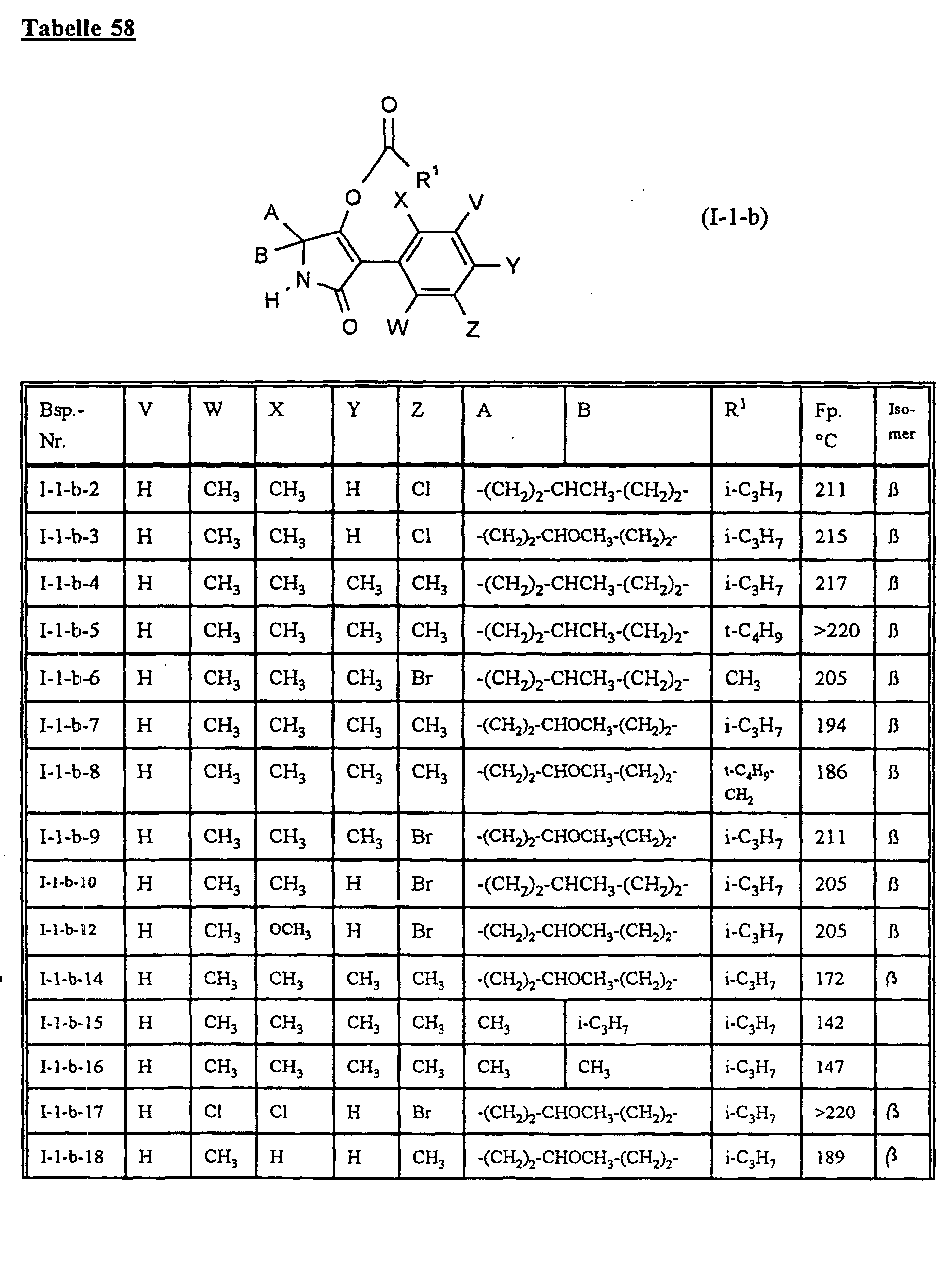

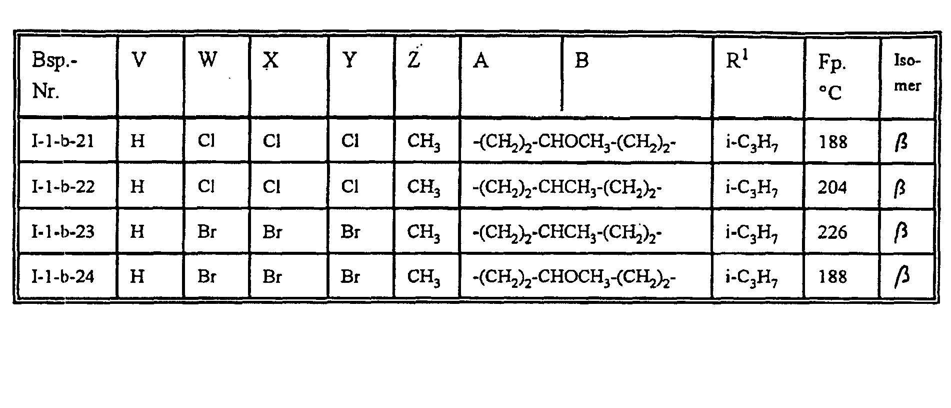

### Beispiel (I-1-c-1)

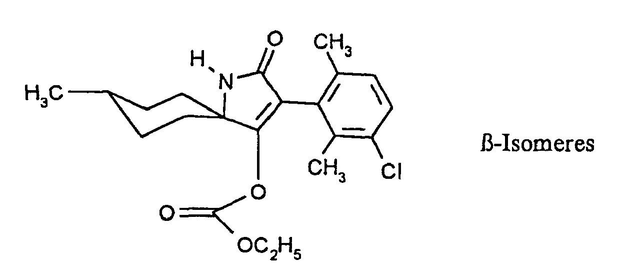

Zu 3,84 g (0,012 Mol) der Verbindung gemäß Beispiel (I-1-a-1) und 1,7 ml (0,012 Mol) Triethylamin in 70 ml wasserfreiem CH₂Cl₂ tropft man bei 0 bis 10°C 1,2 ml (0,012 Mol) Chlorameisensäureethylester in 5 ml wasserfreiem Methylenchlorid und rührt bei Raumtemperatur, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft.
Ausbeute: 3,6 g (76 % der Theorie), Fp.: >220°C.
Analog bzw. gemäß den allgemeinen Angaben zur Herstellung erhält man die folgenden Verbindungen der Formel (I-1-c):

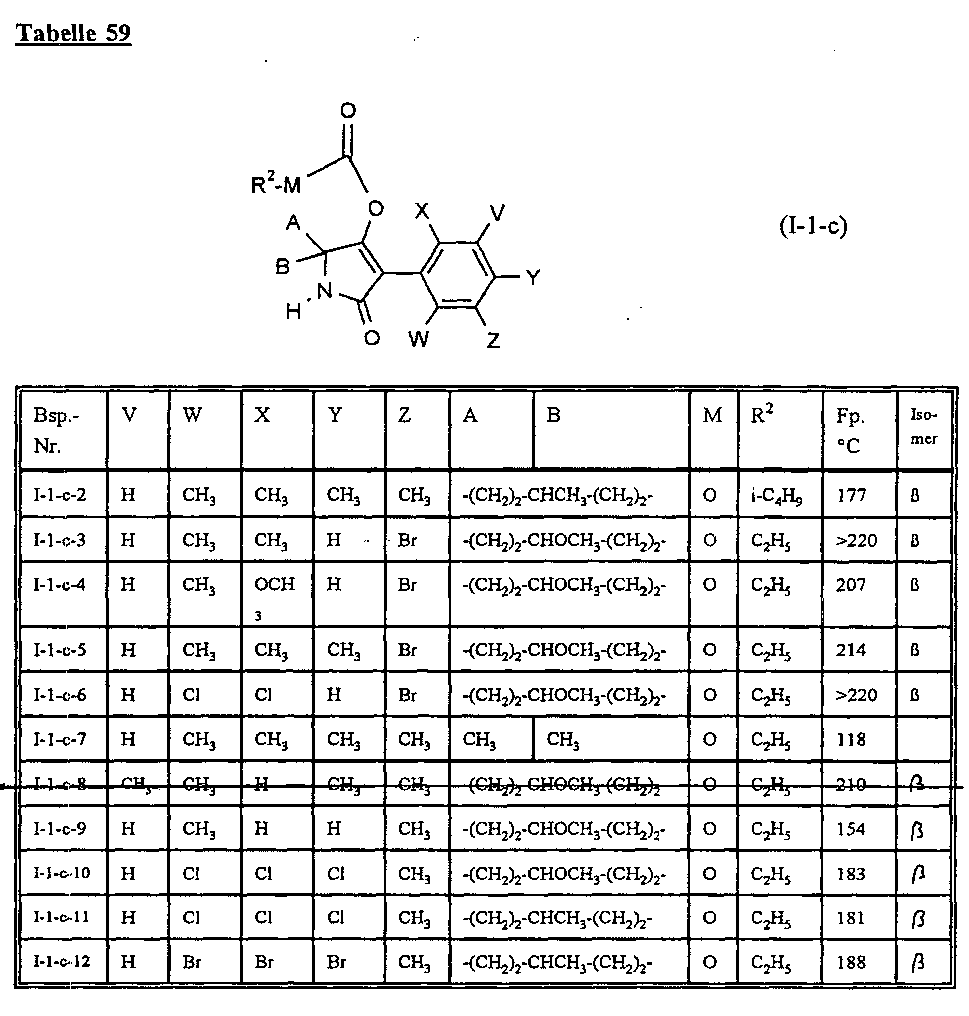

### Beispiel II-1

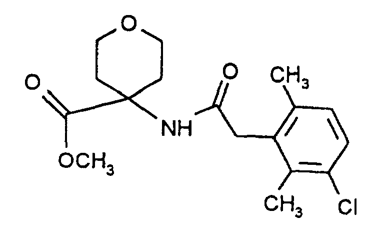

Zu 26,6 g (0,257 Mol) konz. Schwefelsäure tropft man bei 30 bis 40°C vorsichtig 16,7 g (0,0544 Mol) der Verbindung gemäß Beispiel (XXIX-1) in 160 ml wasserfreiem Methylenchlorid und rührt 2 Stunden bei dieser Temperatur. Dann tropft man 37 ml absolutes Methanol so zu, daß sich eine Innentemperatur von etwa 40°C einstellt und rührt noch 6 Stunden bei 40 bis 70°C.

Zur Aufarbeitung gießt man auf 0,28 kg Eis, extrahiert mit Methylenchlorid, wäscht mit wäßriger Natriumhydrogencarbonatlösung, trocknet und dampft ein. Der Rückstand wird aus Methyl-tert.-butylether/n-Hexan umkristallisiert.
Ausbeute: 16,5 g (89 % der Theorie), Fp.: 168°C.

Diese Verbindung ist nicht gegenstand des Anmeldung.

### Beispiel (II-2)

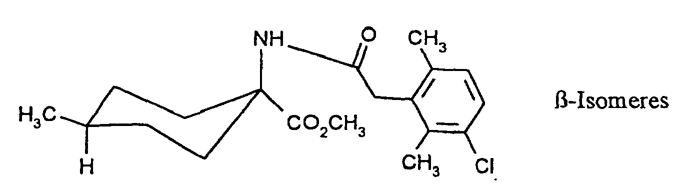

15,9 g (0,08 Mol) 3-Chlor-2,6-dimethylphenylessigsäure und 17,7 ml (0,24 Mol) Thionylchlorid werden 30 Min. bei Raumtemperatur und anschließend bei 80°C gerührt, bis die Gasentwicklung beendet ist. Überschüssiges Thionylchlorid wird bei 50°C im Vakuum entfernt. Dann gibt man 50 ml wasserfreies Toluol zu und dampft erneut ein. Der Rückstand wird in 100 ml wasserfreiem THF aufgenommen (Lösung 1).

Zu 16,8 g cis-4-Methylcyclohexylamin-1-carbonsäuremethylester und 24,6 ml (0,176 Mol) Triethylamin in 160 ml wasserfreiem THF tropft man bei 0 bis 10°C Lösung 1 und rührt anschließend 1 Stunde bei Raumtemperatur. Dann wird abgesaugt, mit wasserfreiem THF gewaschen und eingedampft. Der Rückstand wird in Methylenchlorid aufgenommen, mit 0,5 N HCl gewaschen, getrocknet und eingedampft. Das Rohprodukt wird aus Methyl-tert.-butylether/n-Hexan umkristallisiert.
Ausbeute: 26,9 g (74 % der Theorie), Fp.: 163°C.

Analog zu den Beispielen (II-1) und (II-2) und gemäß den allgemeinen Angaben zur Herstellung werden die folgenden Verbindungen der Formel (II) hergestellt:

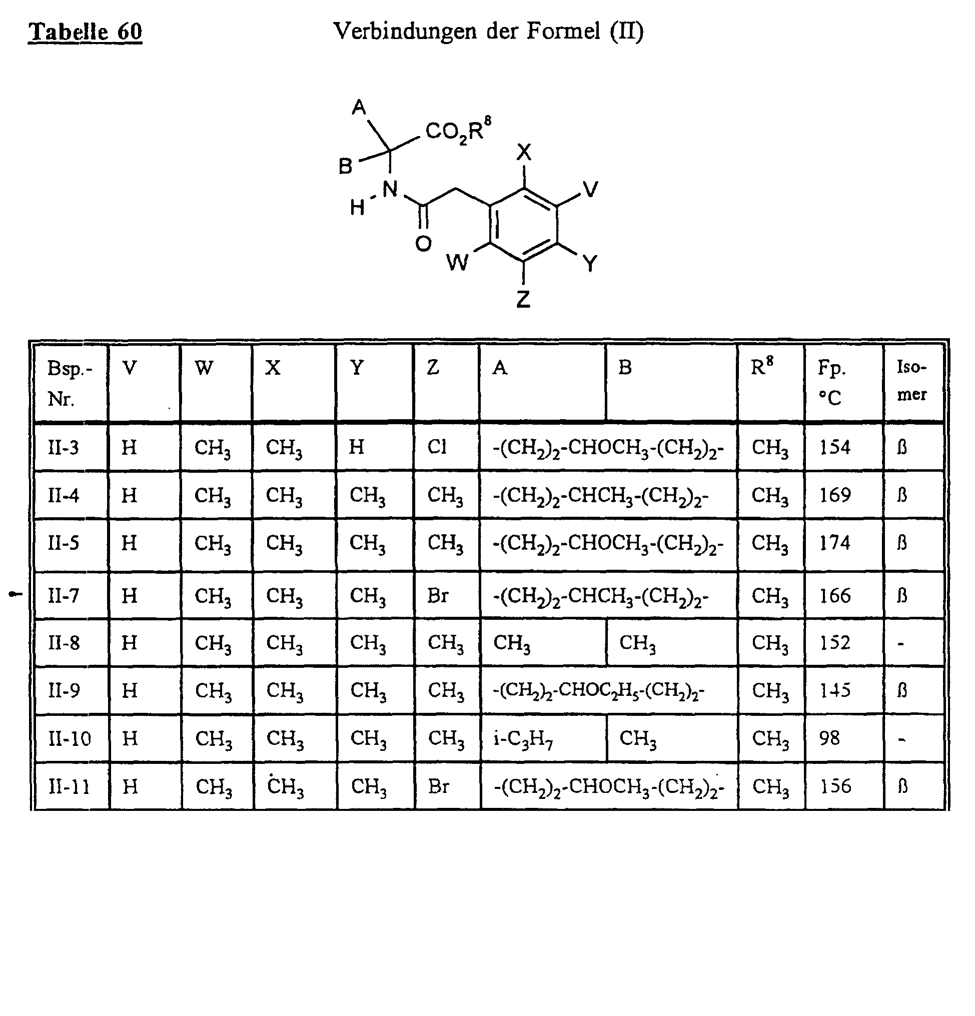

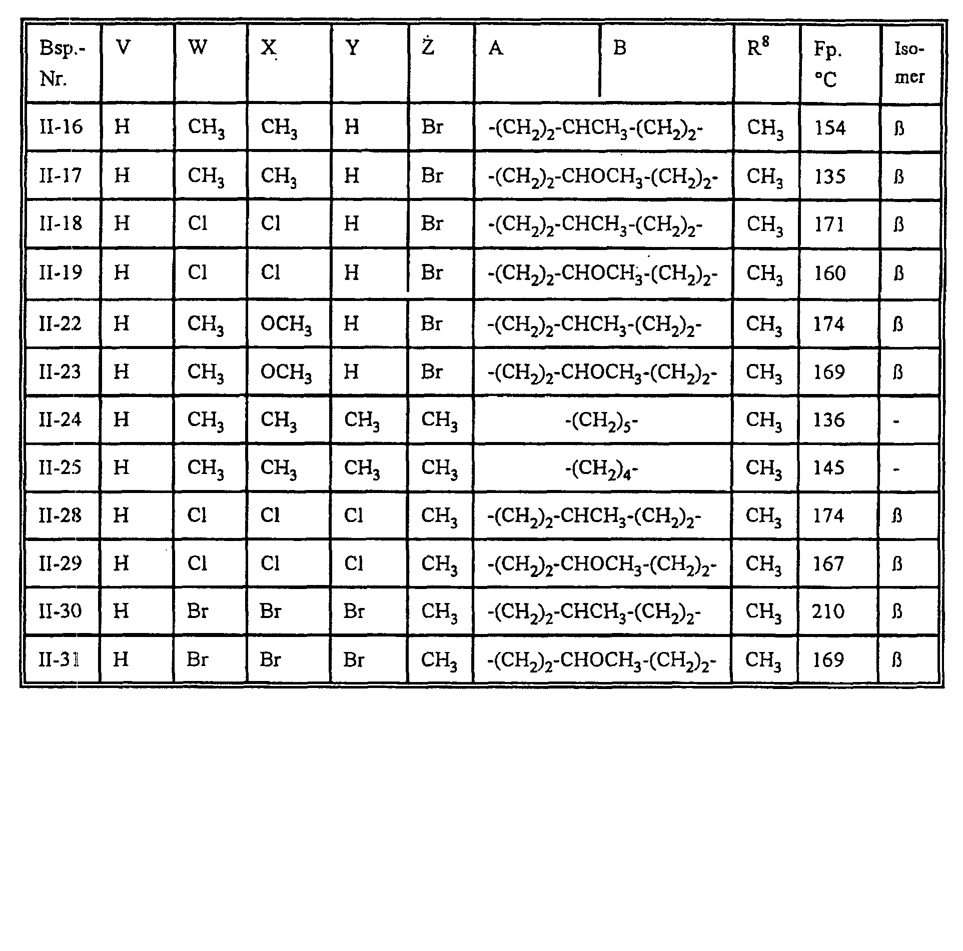

### Beispiel (XXIX-1)

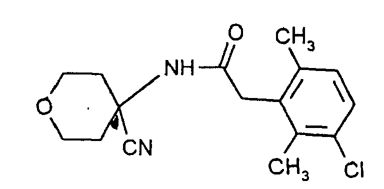

Ausgehend von 12,6 g 3-Chlor-2,6-dimethylphenylessigsäure wird wie in Beispiel (II-2) Lösung 1 hergestellt.

Zu 15,1 g 4-Amino-tetrahydropyran-4-carbonsäurenitril (70 %ig) und 9,2 ml (0,066 Mol) Triethylamin in 120 ml wasserfreiem THF tropft man bei 0 bis 10°C Lösung 1 und rührt noch 1 Stunde bei Raumtemperatur. Dann wird eingedampft und man nimmt den Rückstand in Methylenchlorid auf, wäscht mit 0,5 N HCl, trocknet und dampft ein. Das Rohprodukt wird aus MTB-Ether/n-Hexan umkristallisiert.
Ausbeute: 16,7 g (90 % der Theorie), Fp.: 176°C.

Die Vebindung ist nicht Gegenstand der Anmeldung. Analog zu Beispiel (XXIX-1) und gemäß den allgemeinen Angaben zur Herstellung werden folgende Verbindungen der Formel (XXIX) hergestellt:

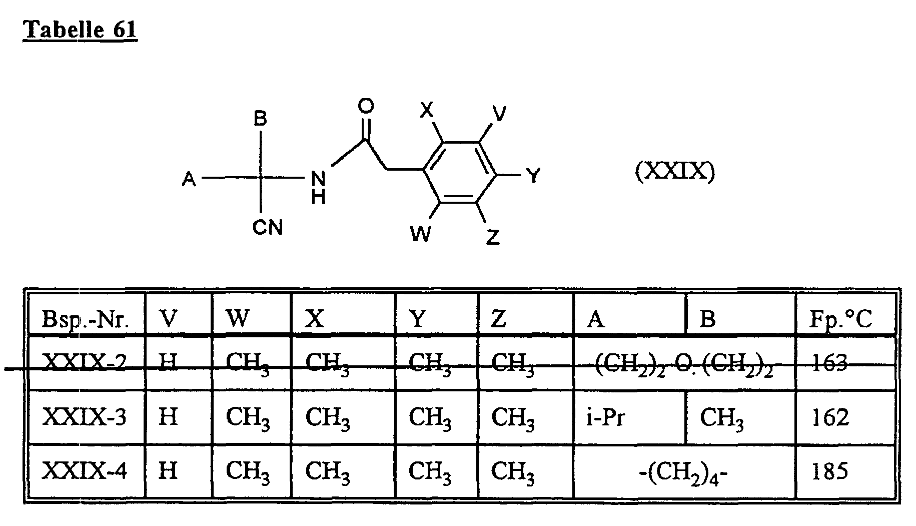

### Beispel (XXXIII-1)

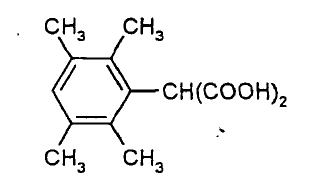

Zu 28,2 g Kaliumhydroxid in 37,8 g Wasser und 75,6 ml Methanol trägt man bei Raumtemperatur 40 g (0,155 Mol) der Verbindung gemäß Beispiel (XXXIV-1) ein und erhitzt 10 Stunden unter Rückfluß.

Nach dem Abkühlen wird im Vakuum eingeengt, der Rückstand mit ca. 100 ml Eiswasser gelöst und unter Kühlung mit halbkonzentrierter Salzsäure angesäuert. Man trennt die Phasen und extrahiert mit Toluol. Der sich abscheidende Feststoff wird abgesaugt, mit wenig Toluol verrührt und erneut abgesaugt.
Ausbeute: 35,20 g (70,3 % der Theorie). Fp.: 193-198°C.

### Beispiel (XXXIII-2)

Analog zu Beispiel (XXXIII-1) bzw. gemäß den allgemeinen Angaben zur Herstellung erhält man die Verbindung 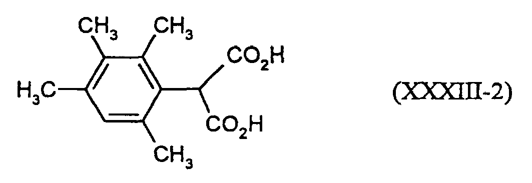
vom Fp.: 181°C (Zersetzung).

### Beispiel (XXIII-1)

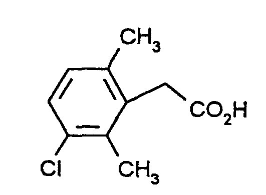

Zu einer Mischung aus 85,8 g (1,532 Mol) KOH in 110,2 ml Wasser und 224 ml Methanol tropft man bei Raumtemperatur 171,9 g der Verbindung gemäß Beispiel XXVI-1 und erhitzt 5 h unter Rückfluß. Nach dem Abkühlen wird mit 300 ml Wasser verdünnt, mit Methyl-tert.-butylether gewaschen. Die wäßrige Phase wird mit halbkonzentrierter Salzsäure angesäuert, abgesaugt, getrocknet und der Rückstand aus Toluol umkristallisiert.
Ausbeute: 111,4 g (Δ 69 % der Theorie) Fp.: 128-130°C.

Analog zu Beispiel (XXIII-1) und gemäß den allgemeinen Angaben zur Herstellung wurden folgende Verbindungen der Formel (XXIII) erhalten:

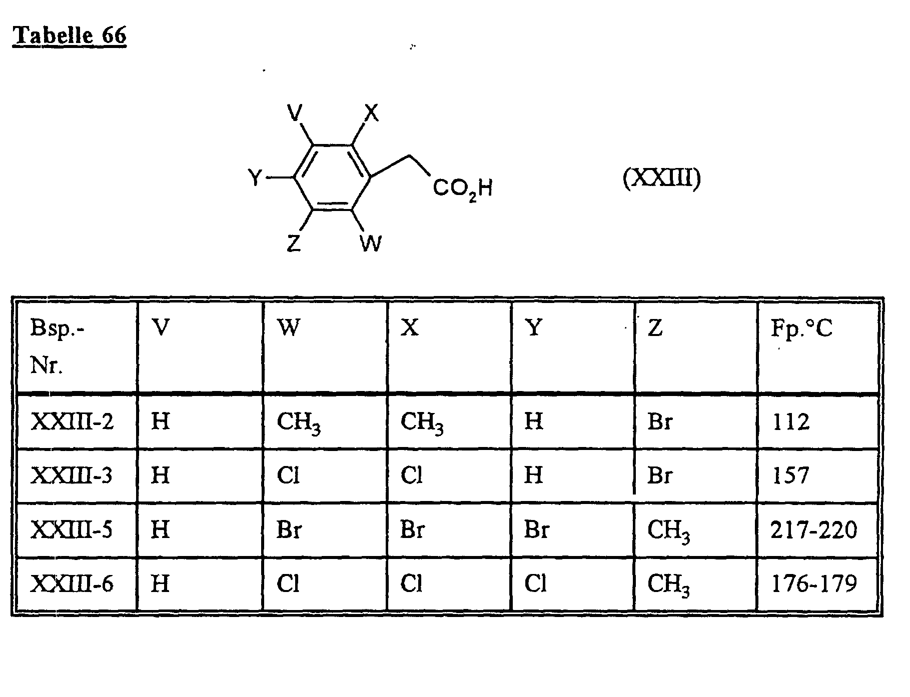

### Beispiel (XXIV-1)

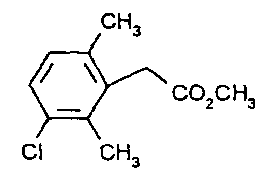

Zu einer Lösung von 353,7 g (1,3 Mol) der Verbindung gemäß Beispiel XXV-1 in 560 ml Methanol tropft man bei Raumtemperatur 992 ml 30 %ige Natriummethylatlösung in Methanol und kocht 5 h unter Rückfluß. Nach Abkühlen auf Raumtemperatur werden 148 ml konz. Schwefelsäure zugetropft, eine Stunde unter Rückfluß erwärmt, abgekühlt, eingeengt, mit Wasser versetzt, mit Methylenchlorid extrahiert, getrocknet und eingeengt. Man erhält 179,1 g eines Öls aus dem gewünschten Produkt XXIV-1 (ca. 51 % nach GC), der Säure XXIII-1 und 1,3-Dichlor-2,6-dimethylbenzol. Für die Umsetzung gemäß Beispiel XXIII-1 wurde das Gemisch verwendet.

Analog zu Beispiel (XXIV-1) und gemäß den allgemeinen Angaben zur Herstellung wurden folgende Verbindungen der Formel (XXIV) erhalten:

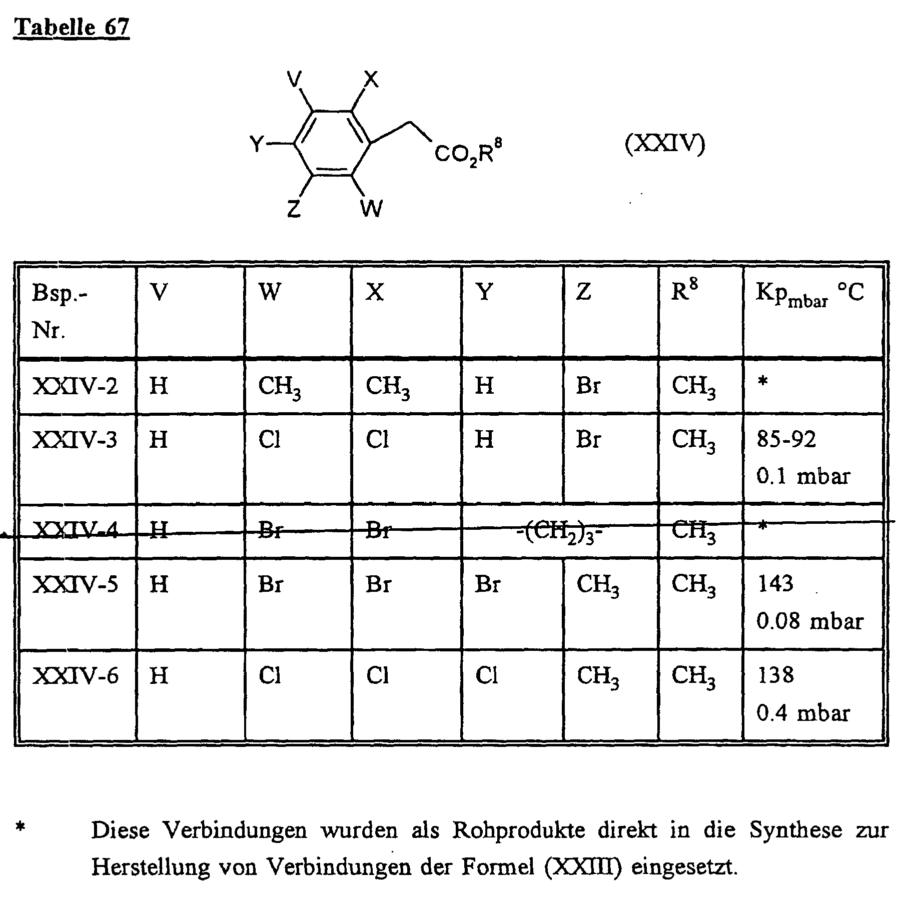

### Beispiel (XXV-1)

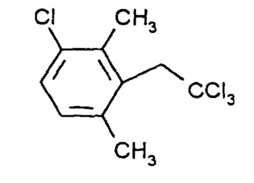

Zu einer gut gekühlten Mischung von 229,7 g (2,27 Mol) tert.-Butylnitrit und 255 g (1,776 Mol) wasserfreiem Kupfer-II-chlorid in 990 ml wasserfreiem Acetonitril tropft man 2205 g (22,8 Mol) 1,1-Dichlorethylen (Vinylidenchlorid), wobei man die Mischung auf Raumtemperatur hält. Dann wird bei einer Temperatur von unter 30°C eine Mischung aus 232 g (1,49 Mol) 3-Chlor-2,6-dimethylanilin in 1500 ml wasserfreiem Acetonitril zugetropft. Es wird bei Raumtemperatur bis zum Ende der Gasentwicklung gerührt, die Mischung wird dann vorsichtig in 6 Liter 20 %ige Salzsäure gegossen und mit Methylenchlorid extrahiert. Die organischen Phasen werden vereint und erneut mit 20 %iger Salzsäure gewaschen, getrocknet und eingeengt. Das zurückbleibende Öl wird ohne weitere Aufarbeitung für die Umsetzung gemäß Beispiel (XXIV-1) verwendet.

Analog zu Beispiel (XXV-1) und gemäß den allgemeinen Angaben zur Herstellung wurden folgende Verbindungen der Formel (XXV) erhalten:

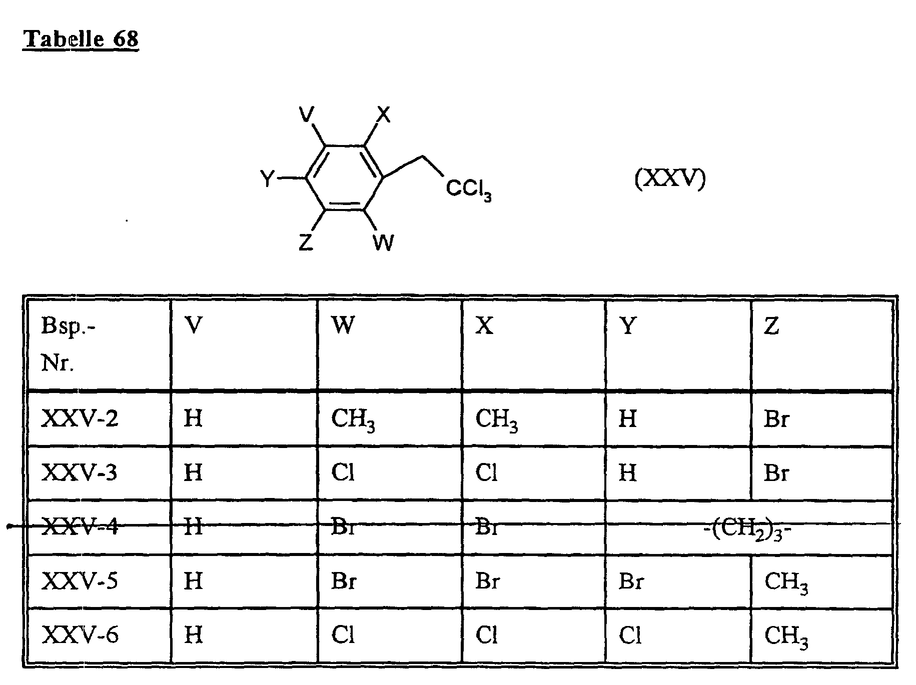

Die in der Tabelle 68 aufgeführten Verbindungen wurden als Rohprodukte in die Verseifung zu den Verbindungen der Formel (XXIV) eingesetzt und deshalb nicht näher charakterisiert.

### Anwendunsgsbeispiele

### Beispiel 1

**Phaedon-Larven-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß Herstellungsbeispielen (I-1-a-2), (I-1-a-3), (I-1-b-1), (I-1-b-2) und (I-1-c-1) bei einer beispielhaften Wirkstofflconzentration von 1000 ppm eine Abtötung von 100 % nach 7 Tagen.

### Beispiel 2

**Tetranychus-Test (OP-resistent/Spritzbehandlung)**

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatte z.B. die Verbindung gemäß Herstellungsbeispiel (I-1-a-2) bei einer beispielhaften Wirkstoffkonzentration von 1000 ppm eine Wirkung von 100 % nach 7 Tagen.

### Beispiel 3

**Plutella-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen (I-1-a-3), (I-1-b-1), (I-1-b-2), (I-1-b-6) und (I-1-c-1) bei einer beispielhaften Wirkstoffkonzentration von 0,1 % eine Abtötung von 100 % nach 7 Tagen.

### Beispiel 4

**Spodoptera-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß Herstellungsbeispielen (I-1-a-2), (I-1-a-3) und (I-1-b-4) bei einer beispielhaften Wirkstoffkonzentration von 0,1 % eine Abtötung von mindestens 80 % nach 7 Tagen.

### Beispiel 5

**Nephotettix-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß Herstellungsbeispielen (I-1-a-1), (I-1-a-2), (I-1-a-3), (I-1-b-1), (I-1-b-2), (I-1-b-3), (I-1-b-4), (I-1-b-5), (I-1-b-6) und (I-1-c-2) bei einer beispielhaften Wirkstoffkonzentration von 0,1 % eine Abtötung von 100 % nach 6 Tagen.

### Beispiel 6

**Myzus-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen (I-1-a-1), (I-1-a-2), (I-1-a-3), (I-1-b-2), (I-1-b-3) (I-2-a-1-) bei einer beispielhaften Wirkstoffkonzentration von 0,1 % eine Abtötung von mindestens 80 % nach 6 Tagen.

### Beispiel 7

**Tetranychus-Test (OP-resistent/Tauchbehandlung)**

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen gemäß den Herstellungsbeispielen (I-1-a-1), (I-1-a-2), (I-1-a-3), (I-1-b-2) und bei einer beispielhaften Wirkstoffkonzentration von 0,1 % eine Abtötung von 100 % nach 5 Tagen.

## Patentansprüche

1. Verbindungen der Formel (I) 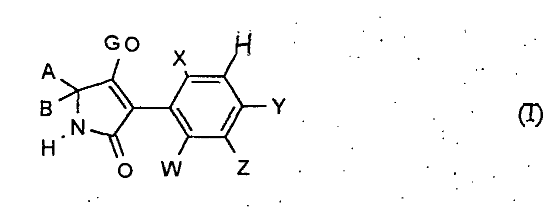 in welcher
W für Halogen oder C₁-C₆-Alkyl, steht
X für Halogen C₁-C₆-Alkyl, oder C₁-C₆-Alkoxy, steht
Y für Wasserstoff, Halogen oder C₁-C₆-Alkyl, steht
Z für Halogen oder C₁-C₆-Alkyl, steht,
A für C₁-C₁₂-Alkyl, steht,
B für C₁-C₁₂-alkyl steht oder
A, B und das Kohlenstoffatom an das sie gebunden sind, für C₃-C₁₀₋Cycloalkyl stehen, welche gegebenenfalls durch C₁-C₈-Alkyl, oder C₁-C₈-Alkoxy, substituiert sind,
G für Wasserstoff (a) oder für eine der Gruppen 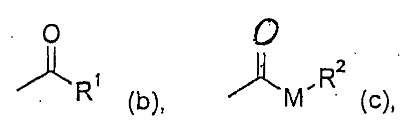 steht,
in welchen
M für Sauerstoff oder Schwefel steht,
R¹ für jeweils gegebenenfalls durch Halogen substituiertes C₁-C₂₀₋Alkyl, C₂-C₂₀-Alkenyl, C₁-C₈-Alkoxy-C₁-C₈-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆₋Alkylthio oder C₁-C₆-Alkylsulfonyl substituiertes Phenyl,
für gegebenenfalls durch Halogen oder C₁-C₆-Alkyl substituiertes 5- oder 6-gliedriges Hetaryl mit ein oder zwei Heteroatomen aus der Reihe Sauerstoff, .Schwefel und Stickstoff,
R₂ für jeweils gegebenenfalls durch Halogen substituiertes C₁-C₂₀-Alkyl, C₂-C₂₀-Alkenyl, C₁-C₈-Alkoxy-C₂-C₈-alkyl,
für jeweils gegebenenfalls durch Halogen, Cyano, Nitro, C₁-C₆₋Alkyl, C₁-C₆-Alkoxy; C₁-C₆-Halogenalkyl oder C₁-C₆-Halogenalkoxy substituiertes Phenyl oder Benzyl steht,

2. Verbindungen der Formel (I) gemäß Anspruch 1, worin
V für Wasserstoff, steht,
W für Fluor, Chlor, Brom oder C₁-₄-Alkyl, steht,
X für Fluor, Chlor, Brom, C₁-₄-Alkyl oder C₁-C₄-Alkoxy, steht,
Y für Wasserstoff, Brom, Chlor oder C₁-C₄-Alkyl, steht
Z für Fluor, Chlor, Brom, oder C₁-C₄-Alkyl, steht,
A für C₁-C₁₀-Alkyl, steht
B für C₁-C₁₀-Alkyl steht
A, B und das Kohlenstoffatom an das sie gebunden sind für C₃-C₈₋Cycloalkyl stehen, welche gegebenenfalls durch C₁-C₆-Alkyl, oder C₁-C₆-Alkoxy, substituiert sind,
D für Wasserstoff steht,
G für Wasserstoff (a) oder für eine der Gruppen 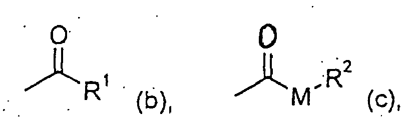
steht, in welchen
M für Sauerstoff oder Schwefel steht.
R¹ für jeweils gegebenenfalls durch Fluor oder Chlor substituiertes C₁₋C₁₆-Alkyl, C₂-C₁₆-Alkenyl, C₁-C₆-Alkoxy-C₁-C₆-alkyl, oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄₋Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für jeweils gegebenenfalls durch Fluor, Chlor, Brom oder C₁-C₄₋Alkyl substituiertes Pyrazolyl, Thiazolyl, Pyridyl, Pyrimidyl, Furanyl oder Thienyl,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl,
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃₋Halogenalkoxy substituiertes Phenyl oder Benzyl steht,

3. Verbindungen der Formel (I) gemäß Anspruch 1, worin
V für Wasserstoff steht,
W für Brom, Chlor oder Methyl steht,
X für Brom, Chlor, Methyl oder Methoxy steht,
Y für Wasserstoff, Brom, Chlor oder Methyl steht,
Z für Chlor, Brom oder Methyl steht,
A für C₁-C₈-Alkyl, steht,
B für C₁-C₈-Alkyl steht, oder
A, B, und das Kohlenstoffatom an das sie gebunden sind für C₃-C₈₋Cycloalkyl stehen, welche gegebenfalls durch Methyl oder Trifluormethyl, Methoxy, Ethoxy, n-Propoxy, substituiert sind
D für Wasserstoff steht,
G für Wasserstoff (a) oder für eine der Gruppen 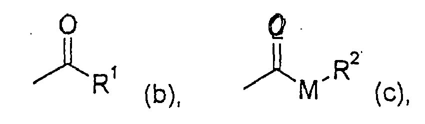
steht, in welchen
M für Sauerstoff oder Schwefel steht.
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für jeweils gegebenenfalls durch Fluor, Chlor, Brom, Methyl oder Ethyl substituiertes Furanyl, Thienyl oder Pyridyl,
Phenoxy-C₁-C₄-alkyl oder
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,

4. Verfahren zur Herstellung von Verbindungen der Formel (I) gemäß Anspruch 1, **dadurch gekennzeichnet daß** man
(A) Verbindungender Formel (I-1-a) 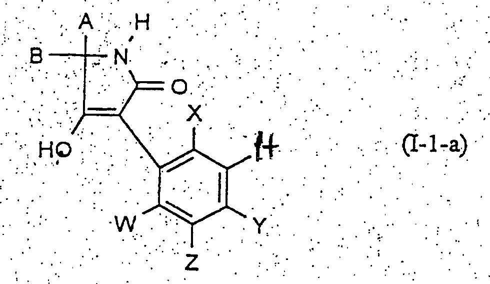
in welcher
A, B, W, X, Y und Z die oben angegebenen Bedeutungen haben,
erhält,
wenn man
Verbindungen der Formel (II) 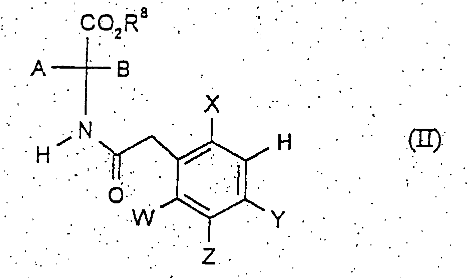
in welcher
A, B, W, X, Y und die oben angegebenen Bedeutungen haben,
und
R⁸ für Alkyl (bevorzugt C₁-C₆ Alkyl) steht,
in Gegenwart eines Verdünnungsmittels und in Gegenwart einer Base intramolekular kondensiert, und gegebenenfalls anschließend die so erhaltenen Verbindungen der Formeln (I-1-a)
(Fα) mit Säurehalogeniden der Formel (VIII) 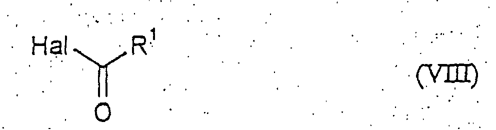
in welcher
R¹ die oben angegebene Bedeutung hat und
Hal für Halogen (insbesondere Chlor oder, Brom) steht
oder
(Fβ) mit Carbonsäureanhy driden der Formel (IX)
R¹-CO-O-CO-R¹ (IX)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(G) mit Chlorameisensäureestern oder Chlorameisensäurethioestern der Formel (X)
R²-M-CO-Cl (X)
in welcher
R² und M die oben angegebenen Bedeutungen haben,
gegebenenfalls in Gegenwart eines Verdünnungsmittels und gegebenenfalls in Gegenwart eines Säurebindemittels umsetzt.

5. Verbindungen der Formel (II) 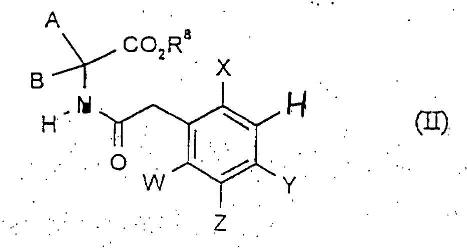
in welcher
A, B, W, X, Y und Z die in Anspruch 1 angegebenen Bedeutungen haben und
R⁸ für Alkyl steht.

6. Verbindungen der Formel (XXI) 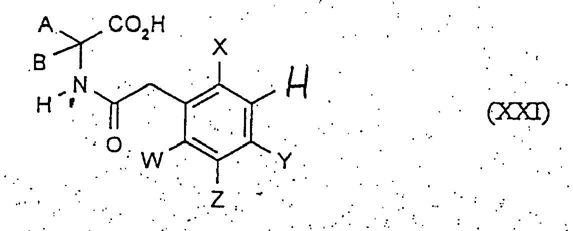
in welcher
A, B, W, X, Y und Z die in Anspruch 1 angegebenen Bedeutungen haben.

7. Verbindungen der Formel (XXIX) 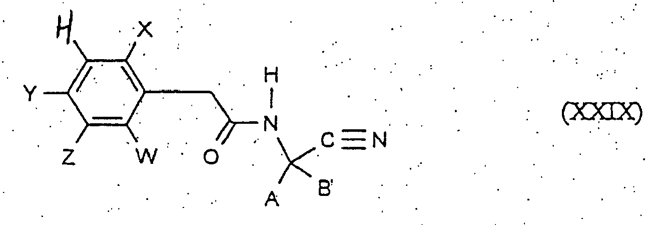
in welcher
A, B, W, X, Y und Z die in Anspruch 1 angegebenen Bedeutungen haben.

8. Verbindungen der Formel (XXIII) 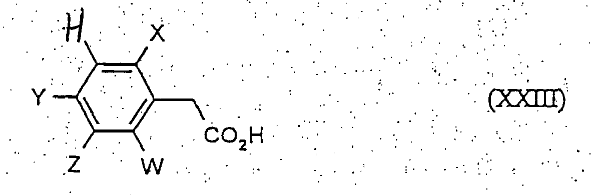 in welcher
W, X, Y und Z die in Anspruch angegebene Bedeutung haben.

9. Verbindungen der Formel (XXIV) 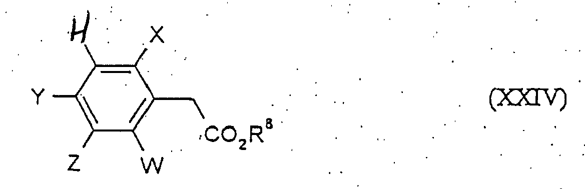
in welcher
W, X, Y und Z die in Anspruch 1 angegebene Bedeutung haben, und
R⁸ für Alkyl steht.

10. Verbindungen der Formel (XXV) 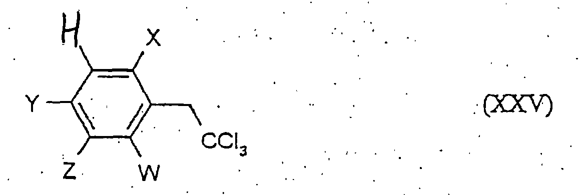
in welcher
W, X, Y und Z die in Anspruch 1 angegebene Bedeutung haben.

11. Schädlingsbekämpfungsmittel oder herbizide Mittel, **ekennzeichnet durch** einen Gehalt an einer Verbindung der Formel (I) gemäß Anspruch 1.

12. Verwendung, von Verbindungen der Formel (I) gemäß Anspruch 1 zur Bekämpfung von Schädlingen und Unkräutern.

13. Verfahren zur Bekämpfung von Schädlingen und Unkräutern, **dadurch gekennzeichnet, daß** man Verbindungen der Formel (I) gemäß Anspruch 1 auf Schädlinge bzw. Unkräuter und/oder ihren Lebensraum einwirken läßt.

14. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 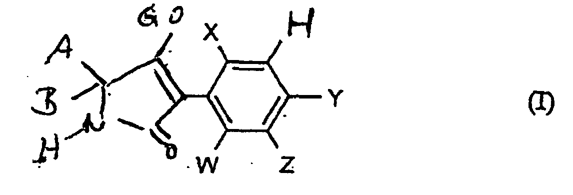 in which
W represents halogen or C₁-C₆-alkyl,
X represents halogen, C₁-C₆-alkyl or C₁-C₆-alkoxy,
Y represents hydrogen, halogen or C₁-C₆-alkyl,
Z represents halogen or C₁-C₆-alkyl,
A represents C₁-C₁₂-alkyl,
B represents C₁-C₁₂-alkyl or
A, B and the carbon atom that they are attached to represent C₃-C₁₀₋cycloalkyl which is optionally substituted by C₁-C₈-alkyl or C₁-C₈₋alkoxy,
G represents hydrogen (a) or one of the groups 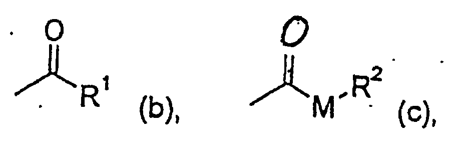
in which
M represents oxygen or sulphur,
R¹ represents respectively optionally halogen-substituted C₁-C₂₀-alkyl, C₂₋C₂₀-alkenyl, C₁-C₈-alkoxy-C₁-C₈-alkyl or respectively halogen-, C₁-C₆₋alkyl- or C₁-C₆-alkoxy-substituted C₃-C₈-cycloalkyl in which one or two not directly adjacent methylene groups are optionally replaced by oxygen and/or sulphur,
optionally halogen-, cyano-, nitro-, C₁-C₆-alkyl-, C₁-C₆-alkoxy-, C₁₋C₆-halogenoalkyl-, C₁-C₆-halogenoalkoxy-, C₁-C₆-alkylthio- or C₁-C₆₋alkylsulphonyl-substituted phenyl,
optionally halogen- or C₁-C₆-alkyl-substituted 5- or 6-membered hetaryl having one or two heteroatoms from the group consisting of oxygen, sulphur and nitrogen,
R² represents respectively optionally halogen-substituted C₁-C₂₀-alkyl, C₂₋C₂₀-alkenyl, C₁-C₈-alkoxy-C₂-C₈-alkyl,
respectively optionally halogen-, cyano-, nitro-, C₁-C₆-aucyl-, C₁-C₆₋alkoxy-, C₁-C₆-halogenoalkyl or C₁-C₆-halogenoalkoxy-substituted phenyl or benzyl.

2. Compounds of the formula (I) according to Claim 1 in which
W represents fluorine, chlorine, bromine or C₁-C₄-alkyl,
X represents fluorine, chlorine, bromine, C₁-C₄-alkyl or C₁-C₄-alkoxy,
Y represents hydrogen, bromine, chlorine or C₁-C₄-alkyl,
Z represents fluorine, chlorine, bromine or C₁-C₄-alkyl,
A represents C₁-C₁₀-alkyl,
B represents C₁-C₁₀-alkyl
A, B and the carbon atom that they are attached to represent C₃-C₈₋cycloalkyl which is optionally substituted by C₁-C₆-alkyl or C₁-C₆₋alkoxy,
D represents hydrogen,
G represents hydrogen (a) or one of the groups 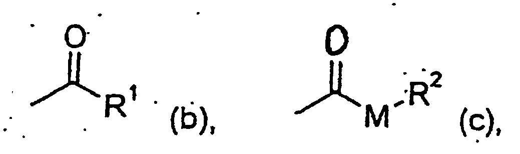
in which
M represents oxygen or sulphur.
R¹ represents respectively optionally fluorine- or chlorine-substituted C₁₋C₁₆-alkyl, C₂-C₁₆-alkenyl, C₁-C₆-alkoxy-C₁-C₆-alkyl, or optionally fluorine-, chlorine-, C₁-C₅-alkyl- or C₁-C₅-alkoxy-substituted C₃-C₇₋cycloalkyl in which one or two not directly adjacent methylene groups are optionally replaced by oxygen and/or sulphur,
optionally fluorine-, chlorine-, bromine-, cyano-, nitro-, C₁-C₄-alkyl-, C₁-C₄-alkoxy-, C₁-C₃-halogenoalkyl-, C₁-C₃-halogenoalkoxy-, C₁-C₄₋alkylthio- or C₁-C₄-alkylsulphonyl-substituted phenyl,
optionally fluorine-, chlorine-, bromine- or C₁-C₄-alkyl-substituted pyrazolyl, thiazolyl, pyridyl, pyrimidyl, furanyl or thienyl,
R² represents respectively optionally fluorine- or chlorine-substituted C₁₋C₁₆-alkyl, C₂-C₁₆-alkenyl, C₁-C₆-alkoxy-C₂-C₆-alkyl,
respectively optionally fluorine-, chlorine-, bromine-, cyano-, nitro-, C₁-C₄-alkyl-, C₁-C₃-alkoxy-, C₁-C₃-halogenoalkyl- or C₁-C₃₋halogenoalkoxy-substituted phenyl or benzyl,

3. Compounds of the formula (I) according to Claim 1 in which
W represents bromine, chlorine or methyl,
X represents bromine, chlorine, methyl or methoxy,
Y represents hydrogen, bromine, chlorine or methyl,
Z represents chlorine, bromine or methyl,
A represents C₁-C₈-alkyl,
B represents C₁-C₈-alkyl or
A, B and the carbon atom that they are attached to represent C₃-C₈₋cycloalkyl which is optionally substituted by methyl or trifluoromethyl, methoxy, ethoxy, n-propoxy,
D represents hydrogen,
G represents hydrogen (a) or one of the groups 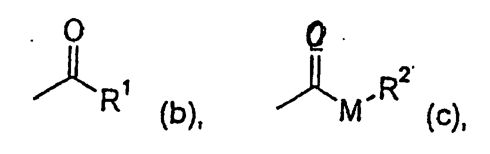
in which
M represents oxygen or sulphur.
R¹ represents respectively optionally fluorine- or chlorine-substituted C₁₋C₁₄-alkyl, C₂-C₁₄-alkenyl, C₁-C₄-alkoxy-C₁-C₆-alkyl or optionally fluorine-, chlorine-, methyl-, ethyl-, n-propyl-, i-propyl-, n-butyl-, i-butyl-, tert-butyl-, methoxy-, ethoxy-, n-propoxy- or isopropoxy-substituted C₃-C₆-cycloalkyl in which one or two not directly adjacent methylene groups are optionally replaced by oxygen and/or sulphur,
optionally fluorine-, chlorine-, bromine-, cyano-, nitro-, methyl-, ethyl-, n-propyl-, i-propyl-, methoxy-, ethoxy-, trifluoromethyl-, trifluoromethoxy-, methylthio-, ethylthio-, methylsulphonyl- or ethylsulphonyl-substituted phenyl,
respectively optionally fluorine-, chlorine-, bromine-, methyl- or ethyl-substituted furanyl, thienyl or pyridyl,
R² represents respectively optionally fluorine- or chlorine-substituted C₁₋C₁₄-alkyl, C₂-C₁₄-alkenyl, CC₄-alkoxy-C₂-C₆-alkyl,
or respectively optionally fluorine-, chlorine-, cyano-, nitro-, methyl-, ethyl-, n-propyl-, i-propyl-, methoxy-, ethoxy-, trifluoromethyl- or trifluoromethoxy-substituted phenyl or benzyl,

4. Process for preparing compounds of the formula (I) according to Claim 1, **characterized in that**
(A) compounds of the formula (I-1-a) 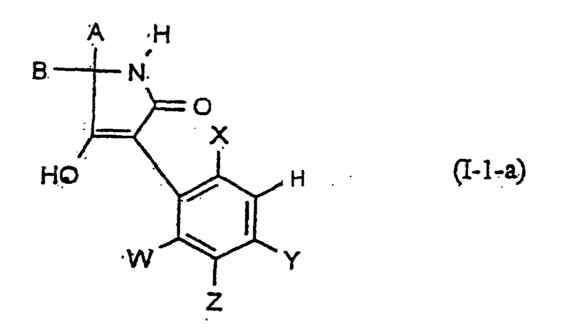
in which
A, B, W, X, Y and Z are each as defined above, are obtained by the intramolecular condensation of
compounds of the formula (II) 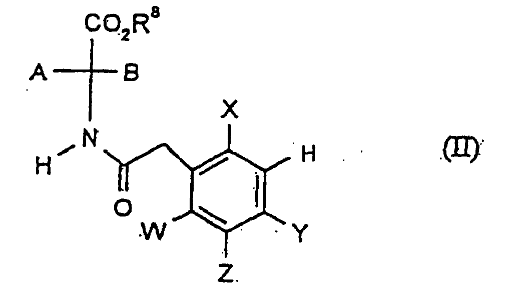
in which,
A, B, W, X, Y and Z are each as defined above
and
R⁸ represents alkyl (preferably C₁-C₆-alkyl)
in the presence of a diluent and in the presence of a base,
and by optionally subsequently reacting the compounds of the formula (I-1-a) obtained in this manner
(Fα) with acid halides of the formula (VIII) 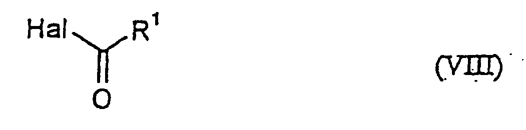
in which
R¹ is as defined above and
Hal represents halogen (in particular chlorine or bromine)
or
(Fβ) with carboxylic anhydrides of the formula (IX)
R¹-CO-O-CO-R¹ (IX)
in which
R¹ is as defined above,
if appropriate in the presence of a diluent and if appropriate in the presence of an acid-binding agent;
or
(G) with chloroformic esters or chloroformic thioesters of the formula (X)
R²-M-CO-Cl (X)
in which
R² and M are each as defined above,
if appropriate in the presence of a diluent and if appropriate in the presence of an acid-binding agent.

5. Compounds of the formula (II) 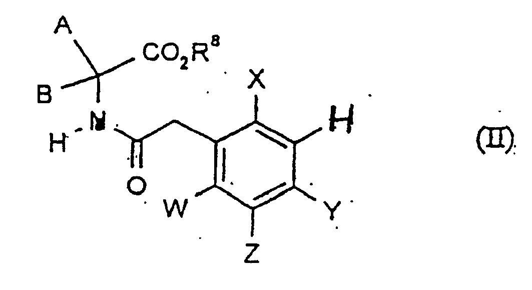
in which
A, B, W, X, Y and Z are each as defined in Claim 1 and
R⁸ represents alkyl.

6. Compounds of the formula (XXI) 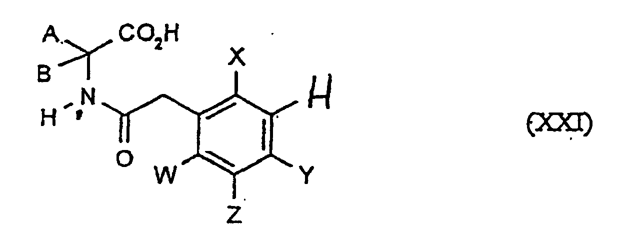
in which
A, B, W, X, Y and Z are each as defined in Claim 1.

7. Compounds of the formula (XXIX) 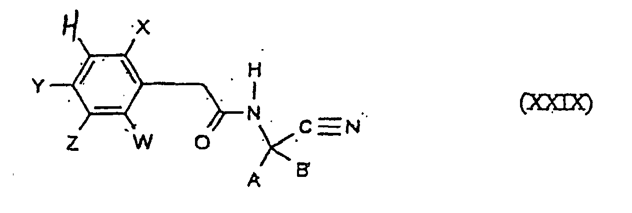
in which
A, B, W, X, Y and Z are each as defined in Claim 1.

8. Compounds of the formula (XXIII) 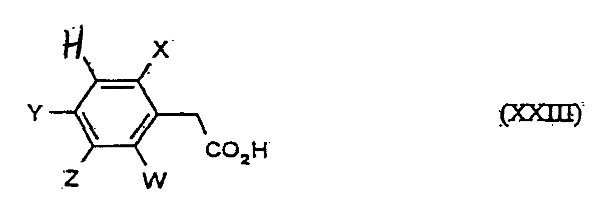
in which
W, X, Y and Z are each as defined in Claim 1.

9. Compounds of the formula (XXIV) 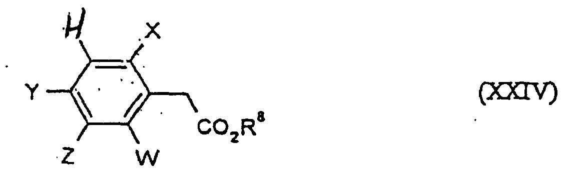
in which
W, X, Y and Z are each as defined in Claim 1 and
R⁸ represents alkyl.

10. Compounds of the formula (XXV) 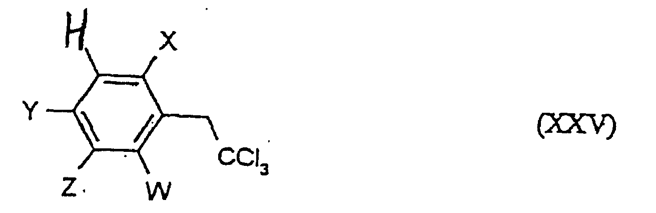
in which
W, X, Y and Z are each as defined in Claim 1.

11. Pesticides or herbicides, **characterized in that** they comprise a compound of the formula (I) according to Claim 1.

12. Use of compounds of the formula (I) according to Claim 1 for controlling pests and weeds.

13. Method for controlling pests and weeds, **characterized in that** compounds of the formula (I) according to Claim 1 are allowed to act on pests and weeds and/or their habitat.

14. Process for preparing pesticides and herbicides, **characterized in that** compounds of the formula (I) according to Claim 1 are mixed with extenders and/or surface-active agents.

## Revendications

1. Composés de la formule (I) : 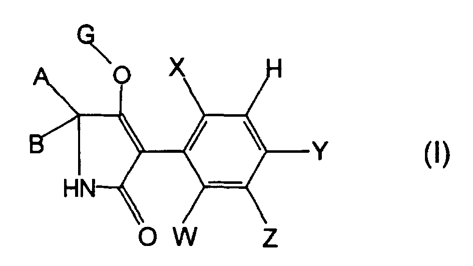
dans laquelle :
W représente un atome d'halogène ou un radical alcoyle en C₁-C₆,
X représente un atome d'halogène, un radical alcoyle en C₁-C₆ ou alcoxy en C₁-C₆,
Y représente l'atome d'hydrogène, un atome d'halogène ou un radical alcoyle en C₁-C₆,
Z représente un atome d'halogène ou un radical alcoyle en C₁-C₆,
A représente un radical alcoyle en C₁-C₁₂,
B représente un radical alcoyle en C₁-C₁₂,
A, B et l'atome de carbone sur lequel ils sont liés, représentent un radical cycloalcoyle en C₃-C₁₀, qui peut être le cas échéant substitué par un radical alcoyle en C₁-C₈ ou alcoxy en C₁-C₈,
G représente l'atome d'hydrogène (a) ou un des restes 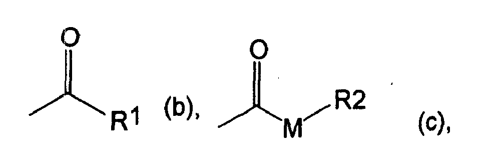
dans lesquels :
M représente l'atome d'oxygène ou de soufre,
R¹ représente un radical alcoyle en C₁-C₂₀, alcényle en C₂-C₂₀, (alcoxy en C₁-C₈) alcoyle en C₁-C₈, chaque fois le cas échéant substitué par un atome d'halogène, ou représente un radical cycloalcoyle en C₃-C₈, le cas échéant substitué par un atome d'halogène, un radical alcoyle en C₁-C₆ ou alcoxy en C₁₋C₆, dans lequel le cas échéant, un ou deux radicaux méthylène non directement voisins sont remplacés par l'atome d'oxygène et/ou de soufre,
représente le radical phényle, le cas échéant substitué par un atome d'halogène, le radical cyano, nitro, un radical alcoyle en C₁-C₆, alcoxy en C₁₋C₆, halogénoalcoyle en C₁-C₆, halogénoalcoxy en C₁-C₆, alcoylthio en C₁-C₆ ou alcoylsulfonyle en C₁-C₆,
représente un radical hétéroaryle à 5 ou 6 membres, avec un ou deux hétéroatomes de la série des atomes d'oxygène, de soufre et d'azote, le cas échéant substitué par un atome d'halogène ou un radical alcoyle en C₁-C₆,
R² représente un radical alcoyle en C₁-C₂₀, alcényle en C₂-C₂₀, (alcoxy en C₁-C₈)alcoyle en C₂-C₈, chaque fois le cas échéant substitué par un atome d'halogène,
représente le radical phényle ou benzyle, chaque fois le cas échéant substitué par un atome d'halogène, le radical cyano, nitro, un radical alcoyle en C₁-C₆, alcoxy en C₁-C₆, halogénoalcoyle en C₁-C₆ ou halogénoalcoxy en C₁-C₆.

2. Composés de la formule (I) suivant la revendication 1, dans lesquels :
W représente l'atome de fluor, de chlore, de brome ou un radical alcoyle en C₁-C₄,
X représente l'atome de fluor, de chlore, de brome, un radical alcoyle en C₁-C₄ ou alcoxy en C₁-C₄,
Y représente l'atome d'hydrogène, l'atome de brome, de chlore ou un radical alcoyle en C₁-C₄,
Z représente l'atome de fluor, de chlore, de brome ou un radical alcoyle en C₁-C₄,
A représente un radical alcoyle en C₁-C₁₀,
B représente un radical alcoyle en C₁-C₁₀,
A, B et l'atome de carbone sur lequel ils sont liés, représentent un radical cycloalcoyle en C₃-C₈, qui peut être le cas échéant substitué par un radical alcoyle en C₁-C₆ ou alcoxy en C₁-C₆,
D représente l'atome d'hydrogène,
G représente l'atome d'hydrogène (a) ou un des restes 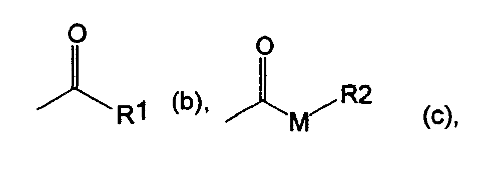
dans lesquels :
M représente l'atome d'oxygène ou de soufre,
R¹ représente un radical alcoyle en C₁-C₁₆, alcényle en C₂-C₁₆, (alcoxy en C₁-C₆)alcoyle en C₁-C₆, chaque fois le cas échéant substitué par l'atome de fluor ou de chlore, ou représente un radical cycloalcoyle en C₃-C₇, le cas échéant substitué par l'atome de fluor ou de chlore, un radical alcoyle en C₁-C₅ ou alcoxy en C₁-C₅, dans lequel le cas échéant, un ou deux radicaux méthylène non directement voisins sont remplacés par l'atome d'oxygène et/ou de soufre,
représente le radical phényle, le cas échéant substitué par l'atome de fluor, de chlore, de brome, le radical cyano, nitro, un radical alcoyle en C₁-C₄, alcoxy en C₁-C₄, halogénoalcoyle en C₁-C₃, halogénoalcoxy en C₁-C₃, alcoylthio en C₁-C₄ ou alcoylsulfonyle en C₁-C₄,
représente le radical pyrazolyle, thiazolyle, pyridyle, pyrimidyle, furannyle ou thiényle le cas échéant substitué par l'atome de fluor, de chlore, de brome ou un radical alcoyle en C₁-C₄,
R² représente un radical alcoyle en C₁-C₁₆, alcényle en C₂-C₁₆, (alcoxy en C₁-C₆)alcoyle en C₂-C₆, chaque fois le cas échéant substitué par l'atome de fluor ou de chlore,
représente le radical phényle ou benzyle, chaque fois le cas échéant substitué par l'atome de fluor, de chlore, de brome, le radical cyano, nitro, un radical alcoyle en C₁-C₄, alcoxy en C₁-C₃, halogénoalcoyle en C₁-C₃ ou halogénoalcoxy en C₁-C₃.

3. Composés de la formule (I) suivant la revendication 1, dans lesquels :
W représente l'atome de brome, de chlore, le radical méthyle,
X représente l'atome de brome, de chlore, le radical méthyle ou méthoxy,
Y représente l'atome d'hydrogène, de brome, de chlore ou le radical méthyle,
Z représente l'atome de chlore, de brome ou le radical méthyle,
A représente un radical alcoyle en C₁-C₈,
B représente un radical alcoyle en C₁-C₈, ou
A, B et l'atome de carbone sur lequel ils sont liés, représentent un radical cycloalcoyle, en C₃-C₈, qui peut être le cas échéant substitué par le radical méthyle, trifluorométhyle, méthoxy, éthoxy, n-propoxy,
D représente l'atome d'hydrogène,
G représente l'atome d'hydrogène (a) ou un des restes 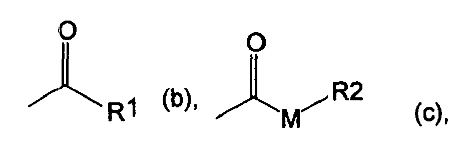
dans lesquels :
M représente l'atome d'oxygène ou de soufre,
R¹ représente un radical alcoyle en C₁-C₁₄, alcényle en C₂-C₁₄, (alcoxy en C₁-C₄)alcoyle en C₁-C₆, chaque fois le cas échéant substitué par l'atome de fluor ou de chlore, ou représente un radical cycloalcoyle en C₃-C₆, le cas échéant substitué par l'atome de fluor, de chlore, le radical méthyle, éthyle, n-propyle, isopropyle, n-butyle, isobutyle, t-butyle, méthoxy, éthoxy, n-propoxy ou isopropoxy, dans lequel le cas échéant, un ou deux radicaux méthylène non directement voisins sont remplacés par l'atome d'oxygène et/ou de soufre,
représente le radical phényle, le cas échéant substitué par l'atome de fluor, de chlore, de brome, le radical cyano, nitro, méthyle, éthyle, n-propyle, isopropyle, méthoxy, éthoxy, trifluorométhyle, triflorométhoxy, méthylthio, éthylthio, méthylsulfonyle ou éthylsulfonyle,
représente le radical furannyle, thiényle ou pyridyle, le cas échéant substitué par l'atome de fluor, de chlore, de brome, le radical méthyle ou éthyle,
R² représente un radical alcoyle en C₁-C₁₄, alcényle en C₂-C₁₄, (alcoxy en C₁-C₉) alcoyle en C₂-C₆, chaque fois le cas échéant substitué par l'atome de fluor ou de chlore,
représente le radical phényle ou benzyle, chaque fois le cas échéant substitué par l'atome de fluor, de chlore, le radical cyano, nitro, le radical méthyle, éthyle, n-propyle, isopropyle, méthoxy, éthoxy, trifluorométhyle, ou triflorométhoxy.

4. Procédé de préparation des composés de la formule (I) suivant la revendication 1, **caractérisé en ce que** :
(A) on obtient des composés de la formule (I-1-a) : 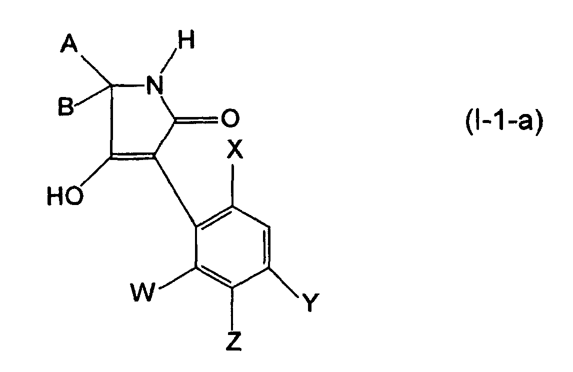
dans laquelle :
A, B, W, X, Y et Z ont les significations indiquées ci-dessus,
lorsque l'on condense de manière intramoléculaire, des composés de la formule (II) : 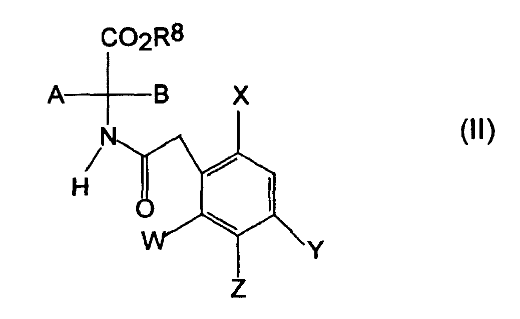
dans laquelle :
A, B, W, X, Y et Z ont les significations indiquées ci-dessus,
et
R⁸ représente un radical alcoyle (de préférence alcoyle en C₁-C₆),
en présence d'un agent de dilution et en présence d'une base,
et ensuite, le cas échéant, les composés (I-1-a) ainsi obtenus
(Fα) sont mis à réagir avec des halogénures d'acide de la formule (VIII) : 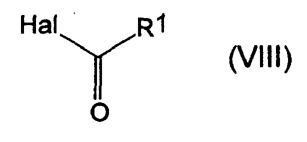
dans laquelle :
R¹ a la signification donnée ci-dessus, et
Hal représente un atome d'halogène (en particulier, chlore ou brome), ou
(Fβ) avec des anhydrides d'acide carboxylique de la formule (IX) :
R¹ - CO - O - CO- - R¹ (IX)
dans laquelle :
R¹ a la signification donnée ci-dessus,
le cas échéant en présence d'un agent de dilution et le cas échéant, en présence d'un agent liant les acides ;
ou
(G) on fait réagir avec des esters de l'acide chloroformique ou des thioesters de l'acide chloroformique de la formule (X) :
R² - M - CO - Cl (X)
dans laquelle :
R² et M ont les significations données ci-dessus,
le cas échéant en présence d'un agent de dilution et le cas échéant, en présence d'un agent liant les acides.

5. Composés de la formule (II) : 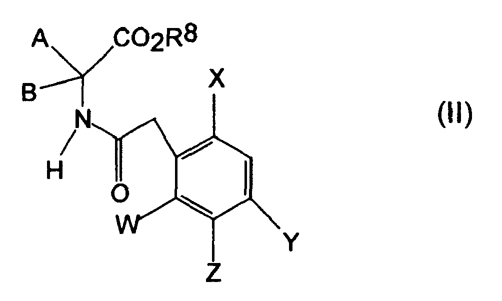
dans laquelle :
A, B, W, X, Y et Z ont les significations indiquées à la revendication 1,
et
R⁸ représente un radical alcoyle.

6. Composés de la formule (XXI) : 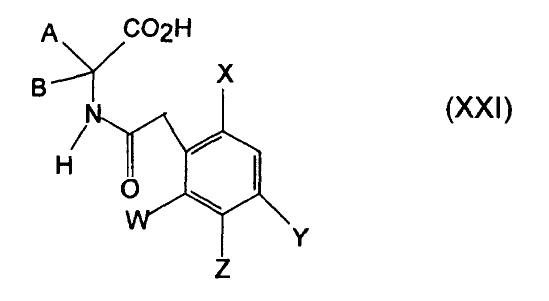
dans laquelle :
A, B, W, X, Y et Z ont les significations indiquées à la revendication 1.

7. Composés de la formule (XXIX) : 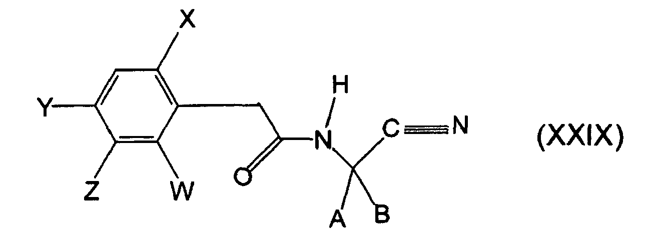
dans laquelle :
A, B, W, X, Y et Z ont les significations indiquées à la revendication 1.

8. Composés de la formule (XXIII) : 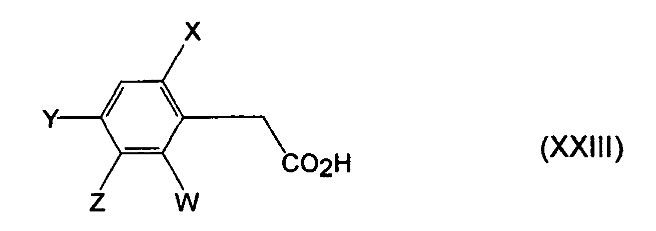
dans laquelle :
W, X, Y et Z ont les significations indiquées à la revendication 1.

9. Composés de la formule (XXIV) : 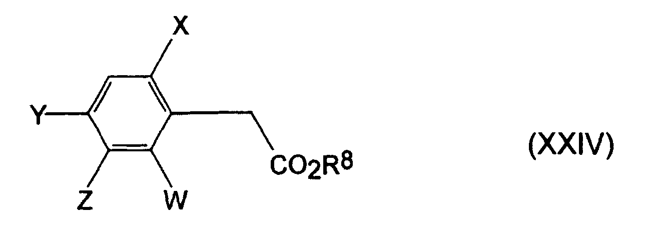
dans laquelle :
W, X, Y et Z ont les significations indiquées à la revendication 1, et.
R⁸ représente un radical alcoyle.

10. Composés de la formule (XXV) : 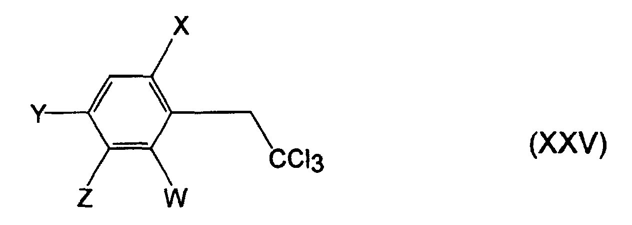
dans laquelle :
W, X, Y et Z ont les significations indiquées à la revendication 1.

11. Agent de lutte contre les parasites ou agent herbicide, **caractérisé par** une teneur en un composé de la formule (I) suivant la revendication 1.

12. Utilisation des composés de la formule (I) suivant la revendication 1, pour lutter contre des parasites et des mauvaises herbes.

13. Procédé pour lutter contre des parasites et des mauvaises herbes, **caractérisé en ce que** l'on fait agir des composés de la formule (I) suivant la revendication 1, sur les parasites ou mauvaises herbes et/ou leur biotope.

14. Procédé de préparation d'agents de lutte contre des parasites et d'agents herbicides, **caractérisé en ce que** l'on mélange des composés de la formule (I) suivant la revendication 1, avec des diluants et/ou agents tensioactifs.
